# EUROPEAN PATENT APPLICATION

(11) **EP 2 107 050 A1**
(43) Date of publication of application: **07.10.2009**
(21) Application number: 08006239.1
(22) Date of filing: 15.06.1994
(51) Int. Cl.: C07C 69/732, C07C 59/42, C07C 69/734, A61K 31/23, C07C 69/736, C07C 69/587

(54) **Lipoxin compounds**

(30) Priority: 15.06.1993 US 77300
(62) Divisional of application: 05026686.5
(71) Applicant: The Brigham and Women's Hospital, Inc., Boston MA 02115 (US)
(72) Inventor: Serhan, Charles, N., Needham, MA 02492 (US)
(74) Representative: Kirkham, Nicholas Andrew

(57) **Abstract**

Compounds having the active site of natural lipoxins, but a longer tissue half-life are disclosed. These small molecules are useful for treating vasoconstrictive, inflammatory, myeloid suppressive, cardiovascular, and gastrointestinal diseases.

## Description

### Government Support

The work leading to this invention was supported in part by one or more grants from the U.S. Government. The U.S. Government therefore may have certain rights in the invention.

### Background

Lipoxins are a group of biologically active mediators derived from arachidonic acid through the action of lipoxygenase enzyme systems. (Serhan, C.N. and Samuelsson, B. (1984) Proc. Natl. Acad. Sci. USA 81:5335). Formation in human cell types is initiated by 5-lipoxygenase or 15-lipoxygenase. (Serhan, C.N. (1991) J. Bioenerg. Biomembr. 23:105). Single-cell types generate lipoxins at nanogram levels during human neutrophil-platelet and eosinophil transcellular biosynthesis of eicosanoids. (Serhan, C.N. and Sheppard, K.-A. (1990) J. Clin. Invest. 85:772). Lipoxins are conjugated tetraene-containing eicosanoids that modulate cellular events in several organ systems.

Lipoxin A₄ (LXA₄) and lipoxin B₄ (LXB₄) are the two major lipoxins. Each enhances protein kinase C (PKC) activity in nuclei of erythroleukemia cells at 10 nM (Beckman, B.S. et al. (1992) Proc. Soc. Exp. Biol. Med. 201:169). Each elicits prompt vasodilation at nM levels (Busija, D.W. et al. (1989) Am. J. Physiol. 256:H468; Katoh, T. et al. (1992) Am. J. Physiol. 263 (Renal Fluid Electrolyte Physiol. 32):F436). The vasodilatory effects of lipoxins are well-documented. For example, administration of LXA₄ in micromolar amounts via inhalation blocks bronchoconstriction in asthmatic patients. (Christie, P.E. et al. (1992) Am. Rev. Respir. Dis. 145:1281).

In the 10⁻¹⁰ M range, LXA₄ also stimulates cell proliferation in combination with suboptimal concentrations of granulocyte-macrophage colony stimulating factor (GM-CSF) to induce myeloid bone marrow colony formation (Stenke, L. et al. (1991) Biochem. Biophys. Res. Commun. 180:255). LXA₄ also stimulates human mononuclear cell colony formation (Popov, G.K. et al. (1989) Bull. Exp. Biol. Med. 107:93).

LXA₄ inhibits chemotaxis of polymorphonuclear leukocytes (Lee, T.H. et al. (1991) Biochem. Biophys. Res. Commun. 180:1416). An equimolar combination of lipoxins has been found to modulate the polymorphonuclear neutrophil-mesangial cell interaction in glomerular inflammation. (Brady, H.R. et al (1990) Am. J. Physiol. 809). Activation of the polymorphonuclear neutrophils (PMN) includes the release of mediators of structural and functional abnormalities associated with the early stages of glomerular inflammation. (Wilson, C.B. and Dixon, F.J. (1986) In: The Kidney, edited by B.M. Brenner and F.C. Rector. Philadelphia, PA: Saunders, p. 800-891).

Lipoxins act as antagonists to leukotrienes (LT), which are mediators of inflammation. LXA₄ modulates LTC₄-induced obstruction of airways in asthmatic patients. (Christie, P.E. et al. (1992) Am. Rev. Respir. Dis. 145:1281). LXA₄ inhibits LTD₄- and LTB₄-mediated inflammation in animal *in vivo* models. (Badr, K.F. et al (1989) Proc. Natl. Acad. Sci. 86:3438; Hedqvist, P. et al. (1989) Acta Physiol. Scand. 137:571). Prior exposure to LXA₄ (nM) blocks renal vasoconstrictor actions of LTD₄ (Katoh, T. et al. (1992) Am. J.Physiol. 263 (Renal Fluid Electrolyte Physiol. 32) F436). Leukotriene-induced inflammation occurs, for example, in arthritis, asthma, various types of shock, hypertension, renal diseases, allergic reactions, and circulatory diseases including myocardial infarction.

Although lipoxins are potent small molecules that could be administered in vivo to treat a number of diseases and conditions, these molecules are short-lived in vivo. Compounds having the same bioactivities as natural lipoxins, but a longer in vivo half-life would be valuable pharmaceuticals.

### Summary of the Invention

This invention features lipoxin analogs, which have an active region that is the same or similar to natural lipoxin, but a metabolic transformation region which is more resistant to in vivo catabolism. The instant disclosed lipoxin analogs therefore have the biological activity of natural lipoxins, but a longer metabolic half-life. Certain of the instant disclosed lipoxin analogs may additionally have an increased *in vivo* potency, higher binding affinity to lipoxin receptors or enhanced bioactivity as compared to natural lipoxins.

The invention also features preferred methods for making the disclosed compounds and intermediates useful in preparation, as well as assays useful for characterizing lipoxin compounds for bioactivity, in vivo half-life, ability to inhibit lipoxin metabolism and/ or for determining binding affinity to a lipoxin receptor.

The instant invention further features lipoxin-based small molecule drugs and their use in treating or preventing a disease or condition associated with an inadequate or inappropriate lipoxin mediated cellular response in a subject. In one embodiment, the lipoxin analog small molecule acts as a leukotriene antagonist and is therefore useful in treating diseases or conditions resulting from leukotriene stimulation such as leukotriene induced inflammations. In another embodiment, the lipoxin analog small molecule initiates osteogenesis and therefore is useful in treating myeloid suppressive disorders.

Like natural lipoxins, the instant disclosed small molecules are highly potent and biocompatible (i.e. non-toxic). However, unlike natural lipoxins, lipoxins analogs inhibit, resist, or more slowly undergo metabolism and therefore have a longer pharmacological activity. Further, the instant disclosed compounds are more lipophilic than natural lipoxins and therefore are more readily taken up by biological membranes.

The invention further relates to diagnostic and research uses of the lipoxin compounds. Additional features and advantages of the invention will become more apparent from the following detailed description and claims.

### Detailed Description of the Invention

As used herein, the following phrases and terms are defined as follows:

A "lipoxin analog" shall mean a compound which has an "active region" that functions like the active region of a "natural lipoxin", but which has a "metabolic transformation region" that differs from natural lipoxin. Lipoxin analogs include compounds which are structurally similar to a natural lipoxin, compounds which share the same receptor recognition site, compounds which share the same or similar lipoxin metabolic transformation region as lipoxin, and compounds which are art-recognized as being analogs of lipoxin. Lipoxin analogs include lipoxin analog metabolites. The compounds disclosed herein may contain one or more centers of asymmetry. Where asymmetric carbon atoms are present, more than one stereoisomer is possible, and all possible isomeric forms are intended to be included within the structural representations shown. Optically active (R) and (S) isomers may be resolved using conventional techniques known to the skilled artisan. The present invention is intended to include the possible diastereiomers as well as the racemic and optically resolved isomers.

The terms "corresponding lipoxin" and "natural lipoxin" refer to a naturally-occurring lipoxin or lipoxin metabolite. Where an analog has activity for a lipoxin-specific receptor, the corresponding or natural lipoxin is the normal ligand for that receptor. For example, where an analog is a LXA₄ analog having specific activity for a LXA₄ specific receptor on differentiated HL-60 cells, the corresponding lipoxin is LXA₄. Where an analog has activity as an antagonist to another compound (such as a leukotriene), which is antagonized by a naturally-occurring lipoxin, that natural lipoxin is the corresponding lipoxin.

"active region" shall mean the region of a natural lipoxin or lipoxin analog, which is associated with in vivo cellular interactions. The active region may bind the "recognition site" of a cellular lipoxin receptor or a macromolecule or complex of macromolecules, including an enzyme and its cofactor. Preferred lipoxin A₄ analogs have an active region comprising C₅-C₁₅ of natural lipoxin A₄. Preferred lipoxin B₄ analogs have an active region comprising C5-C14 of natural lipoxin B4.

The term "recognition site" or receptor is art-recognized and is intended to refer generally to a functional macromolecule or complex of macromolecules with which certain groups of cellular messengers, such as hormones, leukotrienes, and lipoxins, must first interact before the biochemical and physiological responses to those messengers are initiated. As used in this application, a receptor may be isolated, on an intact or permeabilized cell, or in tissue, including an organ. A receptor may be from or in a living subject, or it may be cloned. A receptor may normally exist or it may be induced by a disease state, by an injury, or by artificial means. A compound of this invention may bind reversibly, irreversibly, competitively, noncompetitively, or uncompetitively with respect to the natural substrate of a recognition site.

The term "metabolic transformation region" is intended to refer generally to that portion of a lipoxin, a lipoxin metabolite, or lipoxin analog including a lipoxin analog metabolite, upon which an enzyme or an enzyme and its cofactor attempts to perform one or more metabolic transformations which that enzyme or enzyme and cofactor normally transform on lipoxins. The metabolic transformation region may or may not be susceptible to the transformation. A nonlimiting example of a metabolic transformation region of a lipoxin is a portion of LXA₄ that includes the C-13,14 double bond or the C-15 hydroxyl group, or both.

The term "detectable label molecule" is meant to include fluorescent, phosphorescent, and radiolabeled molecules used to trace, track, or identify the compound or receptor recognition site to which the detectable label molecule is bound. The label molecule may be detected by any of the several methods known in the art.

The term " labeled lipoxin analog" is further understood to encompass compounds which are labeled with radioactive isotopes, such as but not limited to tritium ( ³H), deuterium ( ²H), carbon ( ¹⁴C), or otherwise labeled (e.g. fluorescently). The compounds of this invention may be labeled or derivatized, for example, for kinetic binding experiments, for further elucidating metabolic pathways and enzymatic mechanisms, or for characterization by methods known in the art of analytical chemistry.

The term "inhibits metabolism" means the blocking or reduction of activity of an enzyme which metabolizes a native lipoxin. The blockage or reduction may occur by covalent bonding, by irreversible binding, by reversible binding which has a practical effect of irreversible binding, or by any other means which prevents the enzyme from operating in its usual manner on another lipoxin analog, including a lipoxin analog metabolite, a lipoxin, or a lipoxin metabolite.

The term "resists metabolism" is meant to include failing to undergo one or more of the metabolic degradative transformations by at least one of the enzymes which metabolize lipoxins. Two nonlimiting examples of LXA₄ analog that resists metabolism are 1) a structure which can not be oxidized to the 15-oxo form, and 2) a structure which may be oxidized to the 15-oxo form, but is not susceptible to enzymatic reduction to the 13, 14-dihydro form.

The term "more slowly undergoes metabolism" means having slower reaction kinetics, or requiring more time for the completion of the series of metabolic transformations by one or more of the enzymes which metabolize lipoxin. A nonlimiting example of a LXA₄ analog which more slowly undergoes metabolism is a structure which has a higher transition state energy for C-15 dehydrogenation than does LXA₄ because the analog is sterically hindered at the C-16.

The term "tissue" is intended to include intact cells, blood, blood preparations such as plasma and serum, bones, joints, muscles, smooth muscles, and organs.

The term "halogen" is meant to include fluorine, chlorine, bromine and iodine, or fluoro, chloro, bromo, and iodo.

The term "pharmaceutically acceptable salt" is intended to include art-recognized pharmaceutically acceptable salts. These non-toxic salts are usually hydrolyzed under physiological conditions, and include organic and inorganic bases. Examples of salts include sodium, potassium, calcium, ammonium, copper, and aluminum as well as primary, secondary, and tertiary amines, basic ion exchange resins, purines, piperazine, and the like. The term is further intended to include esters of lower hydrocarbon groups, such as methyl, ethyl, and propyl.

The term "pharmaceutical composition" comprises one or more lipoxin analogs as active ingredient(s), or a pharmaceutically acceptable salt(s) thereof, and may also contain a pharmaceutically acceptable carrier and optionally other therapeutic ingredients. The compositions include compositions suitable for oral, rectal, ophthalmic, pulmonary, nasal, dermal, topical, parenteral (including subcutaneous, intramuscular and intravenous) or inhalation administration. The most suitable route in any particular case will depend on the nature and severity of the conditions being treated and the nature of the active ingredient(s). The compositions may be presented in unit dosage form and prepared by any of the methods well-known in the art of pharmacy. Dosage regimes may be adjusted for the purpose to improving the therapeutic response. For example, several divided dosages may be administered daily or the dose may be proportionally reduced over time. A person skilled in the art normally may determine the effective dosage amount and the appropriate regime. A lipoxin analog pharmaceutic compositions can also refer to combinations comprising lipoxins, lipoxin analogs, and/or lipoxin metabolites, including metabolites of lipoxin analogs. A nonlimiting example of a combination is a mixture comprising a lipoxin analog x which inhibits one enzyme which metabolizes lipoxins and which optionally has specific activity with a lipoxin receptor recognition site, and a second lipoxin analog y which has specific activity with a lipoxin receptor recognition site and which optionally inhibits or resists lipoxin metabolism. This combination results in a longer tissue half-life for at least y since x inhibits one of the enzymes which metabolize lipoxins. Thus, the lipoxin action mediated or antagonized by y is enhanced.

The term "subject" is intended to include living organisms susceptible to conditions or diseases caused or contributed to by inflammation, inflammatory responses, vasoconstriction, and myeloid suppression. Examples of subjects include humans, dogs, cats, cows, goats, and mice. The term subject is further intended to include transgenic species.

### Lipoxin Compounds

The instant invention is based on the surprising finding that lipoxins are rapidly metabolized in a unique fashion by certain cells in vivo. Although other lipoxygenase-derived products (e.g. leukotrienes) are metabolized by ω-oxidation followed by β-oxidation ( Huwyler et al., (1992) Eur.J Biochem. 206, 869-879), the instant invention is based on the unexpected finding that lipoxins are metabolized by a series of oxidation and reduction reactions acting on certain sites of the lipoxin molecule. For example, LXA₄ metabolism has been found to occur, at least in part, via oxidation of the C-15 hydroxyl to generate 15-oxo-LXA₄, reduction of the C-13,14 double bond to yield 13, 14-dihydro-15-oxo-LXA₄ and further reduction to yield 13,14-dihydro-LXA₄. In LXB₄ and its natural isomers the analogous oxidation occurs at the C-5 hydroxyl and reduction occurs at the C-6,7 double bond.

Thus, the instant invention features lipoxin analogs having lipoxin activity, but which are chemically modified to prevent dehydrogenation and therefore subsequent degradation *in vivo.* In these analogs, the C-1 to C-13 portion of the natural lipoxin may or may not be conserved. Variations of the C-1 to C-13 portion include different *cis* or *trans* geometry as well as substitutions. The disclosed compounds is represented below by a structural genus, which is further divided into subgenuses. Subgenuses included in each of the following two R groups is denoted by a Roman numeral on the left of the page.

The instant lipoxins comprising an "active region" and a "metabolic transformation region" as both terms are defined herein are generally of the following structure: 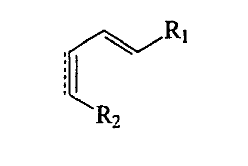 wherein R₁ can be 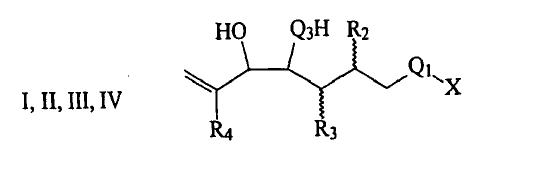 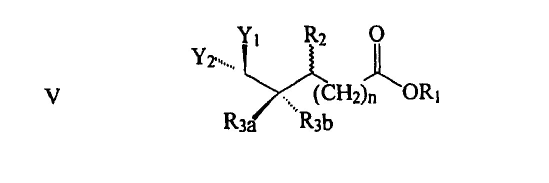 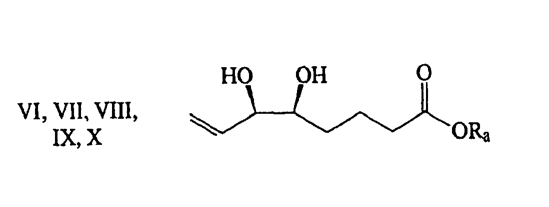 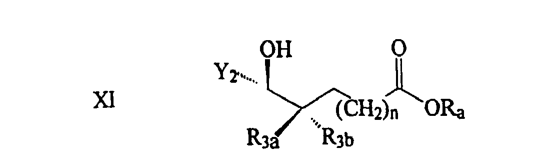 and R₂ can be

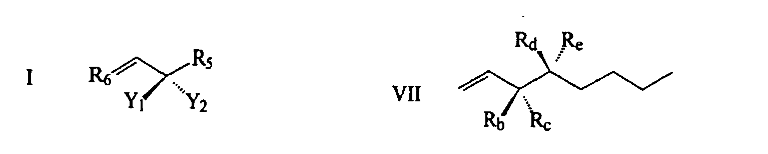

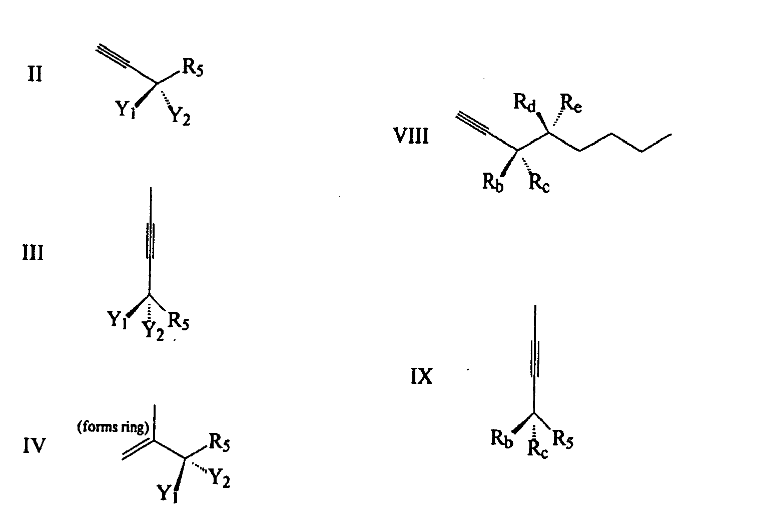

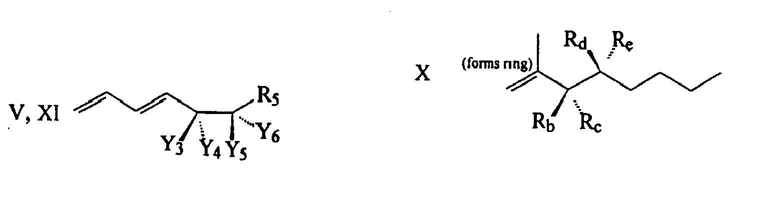

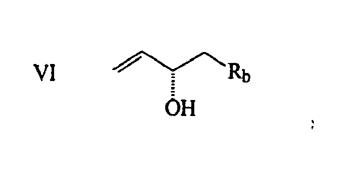

In one embodiment, the lipoxin analogs of this invention have the following structural formula I: 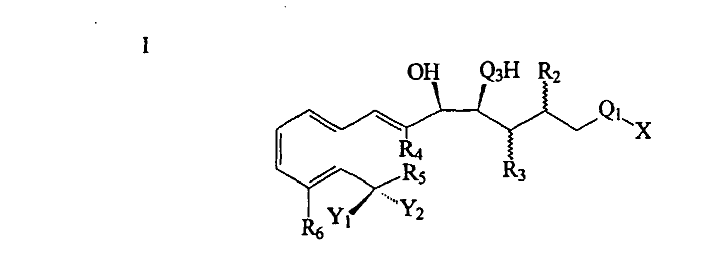 wherein X is R₁, OR₁, or SR₁;
wherein R₁ is
(i) hydrogen;
(ii) alkyl of 1 to 8 carbons atoms, inclusive, which may be straight chain or branched;
(iii) cycloalkyl of 3 to 10 carbon atoms, inclusive;
(iv) aralkyl of 7 to 12 carbon atoms;
(v) phenyl;
(vi) substituted phenyl 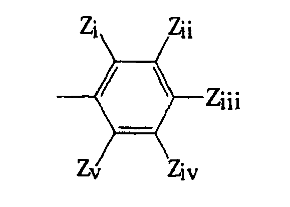 wherein Z ᵢ , Z _{iii,}, and Zᵥ are each independently selected from the group consisting of -NO₂, -CN, -C(=O)-R₁, -SO₃H, and hydrogen;
   wherein Zᵢᵢ and Zᵢᵥ are each independently selected from the group consisting of halogen, methyl, hydrogen, and hydroxyl;
(vii) detectable label molecule; or
(viii) straight or branched chain alkenyl of 2 to 8 carbon atoms, inclusive;
wherein Q₁ is (C=O), SO₂ or (CN);
wherein Q₃ is O, S or NH;
wherein one of R₂ and R₃ is hydrogen and the other is
(a) H;
(b) alkyl of 1 to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -O- or -S-;
   wherein Rₐ is alkylene of 0 to 6 carbons atoms, inclusive, which may be straight chain or branched; and wherein R_{b} is alkyl of 0 to 8 carbon atoms, inclusive, which may be straight chain or branched;
wherein R₄ is
(a) H;
(b) alkyl of 1 to 6 carbon atoms, inclusive, which may be straight chain or branched;
wherein Y₁ or Y₂ is -OH , methyl, or -SH and wherein the other is
(a) H
(b) CHₐZ_{b}
   where a + b =3, a= 0 to 3, b = 0 to 3; and
   Z is cyano, nitro, or halogen;
(c) alkyl of 2 to 4 carbon atoms, inclusive, straight chain or branched; or
(d) alkoxy of 1 to 4 carbon atoms, inclusive;
or Y₁ and Y₂ taken together are
(a) =N; or
(b) =O;
wherein R₅ is
(a) alkyl of 1 to 9 carbon atoms which may be straight chain or branched;
(b)

   -(CH₂)ₙ -Rᵢ

   wherein n = 0 to 4 and Rᵢ is
   (i) cycloalkyl of 3 to 10 carbon atoms, inclusive;
   (ii) phenyl; or
   (iii) substituted phenyl 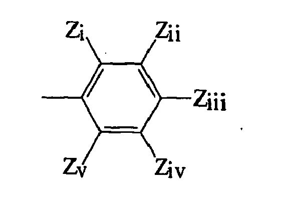 wherein Z ᵢ , Z _{iii,}, and Zᵥ are each independently selected from the group consisting of hydrogen, -NO₂, -CN, - C(=O)-R₁, methoxy, and -SO₃H; and wherein Zᵢᵢ and Zᵢᵥ are each independently selected from the group consisting of halogen, methyl,
      hydrogen, and hydroxyl;
(c)

   -RₐQₐR_{b}

   wherein Qₐ = -O- or -S-;
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched;
(d)

   -C(Rᵢᵢᵢ)(Rᵢᵥ)-R_{¡}

   wherein Rᵢᵢᵢ and Rᵢᵥ are selected independently from the group consisting of
   (i) H;
   (ii) CHₐZ_{b} where a + b = 3, a = 0 to 3, b = 0 + 3, and wherein any Z is independently selected from the group consisting of halogen;
(e) haloalkyl of 1 to 8 carbon atoms, inclusive, and 1 to 6 halogen atoms, inclusive, straight chain or branched; and
wherein R₆ is
(a) H;
(b) alkyl from 1 to 4 carbon atoms, inclusive, straight chain or branched;
(c) halogen; but excluding the C-1 position amides, C-1 position alkanoates, and pharmaceutically acceptable C-1 position salts of (5S, 6R, 15S)-trihydroxy-7E, 9E, 11Z, 13E-eicosatetraenoic acid (LXA₄); and excluding C-5, C-6, and C-15 position alkanoates of LXA₄.

In one embodiment of this invention, the lipoxin analogs have the following structure II: 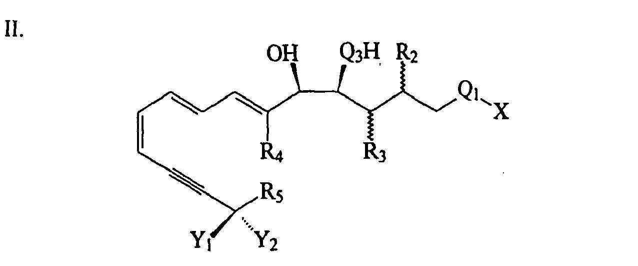 wherein X is R₁, OR₁, or SR₁;
wherein R₁ is
(i) hydrogen;
(ii) alkyl of 1 to 8 carbons atoms, inclusive, which may be straight chain or branched;
(iii) cycloalkyl of 3 to 10 carbon atoms, inclusive;
(iv) aralkyl of 7 to 12 carbon atoms; (v) phenyl;
(vi) substituted phenyl 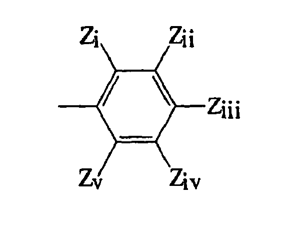 wherein Zᵢ, Z_{iii,}, and Zᵥ are each independently selected from the group consisting of -NO₂, -CN, -C(=O)-R₁, hydrogen, and -SO₃H;
   wherein Zᵢᵢ and Zᵢᵥ are each independently selected from the group consisting of halogen, methyl, hydrogen, and hydroxyl;
(vii) detectable label molecule, such as but not limited to fluorescent labels; or
(viii) alkenyl of 2 to 8 carbon atoms, inclusive, straight chain or branched;
wherein Q₁ is (C=O), SO₂ or (C=N);
wherein. Q₃ is O, S or NH;
wherein one of R₂ and R₃ is hydrogen and the other is
(a) H;
(b) alkyl of I to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -O- or -S-;
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched;
wherein R₄ is
(a) H;
(b) alkyl of 1 to 6 carbon atoms, inclusive, which may be straight chain or branched;
wherein Y₁ or Y₂ is -OH , methyl, -H or -SH and wherein the other is
(a) H;
(b) CHₐZ_{b}
   where a + b =3, a= 0 to 3, b = 0 to 3
   Z is cyano, nitro, or halogen including F, Cl, Br, I;
(c) alkyl of 2 to 4 carbon atoms, inclusive, straight chain or branched;
(d) alkoxy of 1 to 4 carbon atoms, inclusive;
or Y₁ and Y₂ taken together are
(a) =N; or
(b) =O;
wherein R₅ is
(a) alkyl of 1 to 9 carbon atoms which may be straight chain or branched;
(b)

   -(CH₂)ₙ -R_{¡}

   wherein n = 0 to 4 and Rᵢ is
   (i) cycloalkyl of 3 to 10 carbon atoms, inclusive;
   (ii) phenyl; or
   (iii) substituted phenyl 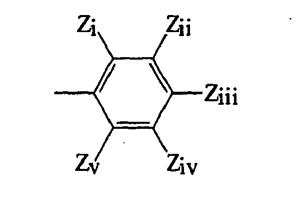 wherein Z ᵢ , Z _{iii,}, and Zᵥ are each independently selected from the group consisting of hydrogen, -NO₂, -CN, - C(=O)-R₁, methoxy, and -SO₃H; wherein Zᵢᵢ and Zᵢᵥ are each independently selected from the group consisting of halogen, methyl, hydrogen, and hydroxyl;
(c)

   -RₐQₐR_{b}

   wherein Qₐ = -O- or -S-; and
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched;
(d)

   -C(Rᵢᵢᵢ)(Rᵢᵥ)-Rᵢ

   wherein Rᵢᵢᵢ and Rᵢᵥ are selected independently from the group consisting of
   (i) H; and
   (ii) CHₐZ_{b} where a + b = 3, a = 0 to 3, b = 0 + 3 wherein any Z is selected from the group consisting of halogen.
(e) haloalkyl of 1 to 8 carbon atoms, inclusive, and 1 to 6 halogen atoms, inclusive, straight chain or branched.

In one embodiment of this invention, the lipoxin analogs have the following structure III: 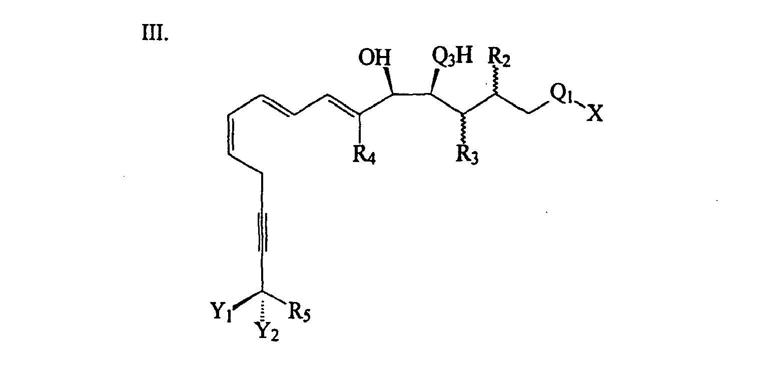 wherein X is R₁, OR₁, or SR₁;
wherein R₁ is
(i) hydrogen;
(ii) alkyl of 1 to 8 carbons atoms, inclusive, which may be straight chain or branched;
(iii) cycloalkyl of 3 to 10 carbon atoms, inclusive;
(iv) aralkyl of 7 to 12 carbon atoms;
(v) phenyl;
(vi) substituted phenyl 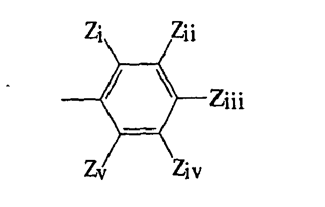 wherein Zᵢ, Z_{iii,}, and Zᵥ are each independently selected from the group consisting of -NO_{2,} -CN, -C(=O)-R₁, hydrogen, and -SO₃H;
   wherein Zᵢᵢ and Zᵢᵥ are each independently selected from the group consisting of halogen, methyl, hydrogen, and hydroxyl;
(vii) detectable label molecule; or
(viii) alkenyl of 2 to 8 carbon atoms, inclusive, straight chain or branched;
wherein Q₁ is (C=O), SO₂ or (C=N);
wherein Q₃ is O, S or NH;
wherein one of R₂ and R₃ is hydrogen and the other is
(a) H;
(b) alkyl of 1 to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -O- or -S-;
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched;
wherein R₄ is
(a) H; or
(b) alkyl of 1 to 6 carbon atoms, inclusive, which may be straight chain or branched;
wherein Y₁ or Y₂ is hydroxyl , methyl, hydrogen or thiol and
wherein the other is
(a) H;
(b) CHₐZ_{b}
   where a + b =3, a= 0 to 3, b = 0 to 3
   Z is cyano, nitro, or halogen [including F, Cl, Br, I];
(c) alkyl of 2 to 4 carbon atoms, inclusive, straight chain or branched;
(d) alkoxy of 1 to 4 carbon atoms, inclusive;
or Y₁ and Y₂ taken together are
(a) =N; or
(b) =O; and
wherein R₅ is
(a) alkyl of 1 to 9 carbon atoms which may be straight chain or branched;
(b)

   -(CH₂)ₙ -R_{¡}

   wherein n = 0 to 4 and Rᵢ is
   (i) cycloalkyl of 3 to 10 carbon atoms, inclusive;
   (ii) phenyl;
   (iii) substituted phenyl 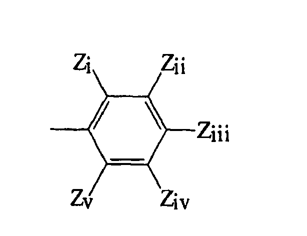 wherein Z ᵢ , Z _{iii,}, and Zᵥ are each independently selected from the group consisting of hydrogen, -NO₂, -CN, -C(=O)-R₁, methoxy, and -SO₃H;
      wherein Zᵢᵢ and Zᵢᵥ are each independently selected from the group consisting of halogen, methyl, hydrogen, and hydroxyl;
(c)

   -RₐQₐB_{b}

   wherein Qₐ = -O- or -S-;
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched; or
(d)

   -C(Rᵢᵢᵢ)(Rᵢᵥ)-R_{¡}

   wherein Rᵢᵢᵢ and Rᵢᵥ are selected independently from the group consisting of
   (i) H;
   (ii) CHₐZ_{b} where a + b = 3, a = 0 to 3, b = 0 + 3
   wherein any Z is selected from the group consisting of halogen.

In another embodiment of this invention, lipoxin analogs have the following structural formula IV: 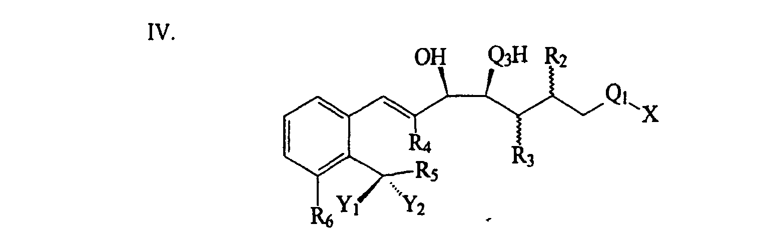 wherein X is R₁, OR₁, or SR₁;
wherein R₁ is
(i) hydrogen;
(ii) alkyl of 1 to 8 carbons atoms, inclusive, which may be straight chain or branched;
(iii) cycloalkyl of 3 to 10 carbon atoms, inclusive;
(iv) aralkyl of 7 to 12 carbon atoms; (v) phenyl;
(vi) substituted phenyl 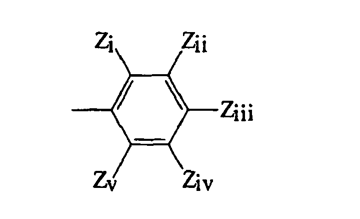 wherein Zᵢ, Z_{iii,}, and Zᵥ are each independently selected from the group consisting of -NO₂, -CN, -C(=O)-R₁, methoxy, hydrogen, and -SO₃H;
   wherein Zᵢᵢ and Zᵢᵥ are each independently selected from the group consisting of halogen, methyl, hydrogen, and hydroxyl;
(vii) detectable label molecule; or
(viii) alkenyl of 2 to 8 carbon atoms, inclusive, straight chain or branched;
wherein Q₁ is (C=O), SO₂ or (CN);
wherein Q₃ is O, S or NH;
wherein one of R₂ and R₃ is hydrogen and the other is
(a) H;
(b) alkyl of 1 to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -O- or -S-;
   wherein Rₐ is alkylene of 0 to 6 carbons atoms, inclusive, which may be straight chain or branched;
   wherein R_{b} is alkyl of 0 to 8 carbon atoms, inclusive, which may be straight chain or branched;
wherein R₄ is
(a) H; or
(b) alkyl of 1 to 6 carbon atoms, inclusive, which may be straight chain or branched;
wherein Y₁ or Y₂ is -OH, methyl, or -SH and wherein the other is
(a) H;
(b) CHₐZ_{b}
   where a + b =3, a= 0 to 3, b = 0 to 3, Z is cyano, nitro, or halogen;
(c) alkyl of 2 to 4 carbon atoms, inclusive, straight chain or branched; or
(d) alkoxy of 1 to 4 carbon atoms, inclusive;
or Y₁ and Y₂ taken together are
(a) =N; or
(b) =O;
wherein R₅ is
(a) alkyl of 1 to 9 carbon atoms which may be straight chain or branched;
(b)

   -(CH₂)ₙ -R_{¡}

   wherein n = 0 to 4 and Rᵢ is
   (i) cycloalkyl of 3 to 10 carbon atoms, inclusive;
   (ii) phenyl; or
   (iii) substituted phenyl 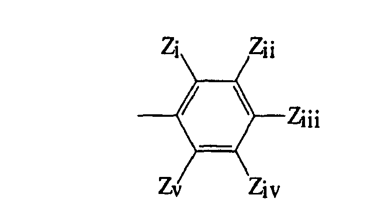 wherein Z ᵢ , Z _{iii,}, and Zᵥ are each independently selected from the group consisting of hydrogen, -NO₂, -CN, -C(=O)-R₁, methoxy, and -SO₃H;
      wherein Zᵢᵢ and Zᵢᵥ are each independently selected from the group consisting of halogen, methyl, hydrogen, and hydroxyl;
(c) RₐQₐR_{b}
   wherein Qₐ = -O- or -S-;
   wherein Rₐ is alkylene of 0 to 6 carbons atoms, inclusive, which may be straight chain
   or branched;
   wherein R_{b} is alkyl of 0 to 8 carbon atoms, inclusive, which may be straight chain or branched;
(d)

   -C(Rᵢᵢᵢ)(Rᵢᵥ)-Rᵢ

   wherein Rᵢᵢᵢ and Rᵢᵥ are selected independently from the group consisting of
   (i) H; or
   (ii) CHₐZ_{b} where a + b = 3, a = 0 to 3, b = 0 + 3, and
      wherein any Z is selected from the group consisting of halogen; or
(e) haloalkyl of 1 to 8 carbon atoms, inclusive, and 1 to 6 halogen atoms, inclusive, straight chain or branched; and
wherein R₆ is
(a) H;
(b) alkyl from 1 to 4 carbon atoms, inclusive, straight chain or branched; or
(c) halogen.

In another embodiment of this invention, lipoxin analogs have the following structural formula V: 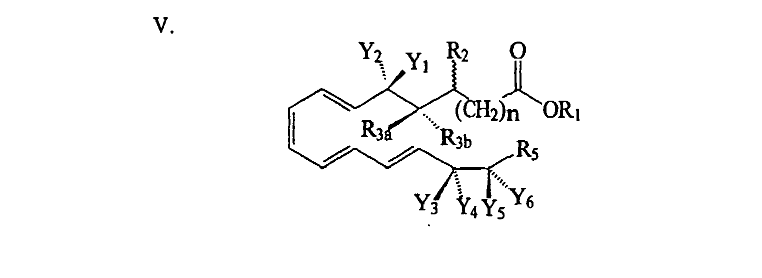 wherein R₁ is
(i) hydrogen;
(ii) alkyl of 1 to 8 carbons atoms, inclusive, which may be straight chain or branched;
(iii) cycloalkyl of 3 to 10 carbon atoms, inclusive;
(iv) aralkyl of 7 to 12 carbon atoms;
(v) phenyl;
(vi) substituted phenyl 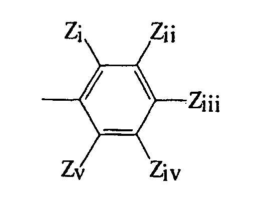 wherein Z ᵢ , Z _{iii,}, and Zᵥ are each independently selected from the group consisting of -NO₂, -CN, -C(=O)-R₁, hydrogen, and -SO₃H;
   wherein Zᵢᵢ and Zᵢᵥ are each independently selected from the group consisting of halogen, methyl, hydrogen, and hydroxyl;
(vii) detectable label molecule; or
(viii) alkenyl of 2 to 8 carbon atoms, inclusive, straight chain or branched;
wherein n = 1 to 10, inclusive;
wherein R₂, R₃ₐ, and R_{3b} are independently selected from
(a) H;
(b) alkyl of 1 to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -O- or -S-;
   wherein Rₐ is alkylene of 0 to 6 carbons atoms, inclusive, which may be straight chain or branched; and wherein R_{b} is alkyl of 0 to 8 carbon atoms, inclusive, which may be straight chain or branched;
wherein Y₁ or Y₂ is -OH , methyl, hydrogen, or -SH and
wherein the other is
(a) H;
(b) CHₐZ_{b}
   where a + b =3, a= 0 to 3, b = 0 to 3, and
   Z is cyano, nitro, or halogen;
(c) alkyl of 2 to 4 carbon atoms, inclusive, straight chain or branched;
(d) alkoxy of 1 to 4 carbon atoms, inclusive, straight chain or branched;
or Y₁ and Y₂ taken together are
(a) =N; or
(b) =O;
wherein Y₃ or Y₄ is -OH , methyl, hydrogen, or -SH and
wherein the other is
(a) H;
(b) CHₐZ_{b}
   wherein a + b =3, a= 0 to 3, b = 0 to 3,
   and any Z is cyano, nitro, or halogen;
(c) alkyl of 2 to 4 carbon atoms, inclusive, straight chain or branched;
(d) alkoxy of 1 to 4 carbon atoms, inclusive, straight chain or branched;
or Y₃ and Y₄ taken together are
(a) =N; or
(b) =O;
wherein Y₅ or Y₆ is -OH , methyl, hydrogen, or -SH and
wherein the other is
(a) H;
(b) CHₐZ_{b}
   where a + b =3, a= 0 to 3, b = 0 to 3
   Z is cyano, nitro, or halogen;
(c) alkyl of 2 to 4 carbon atoms, inclusive, straight chain or branched;
(d) alkoxy of I to 4 carbon atoms, inclusive, straight chain or branched;
or Y₅ and Y₆ taken together are
(a) =N; or
(b) =O;
wherein R₅ is
(a) alkyl of 1 to 9 carbon atoms which may be straight chain or branched;
(b)

   -(CH₂)ₙ -Rᵢ

   wherein n = 0 to 4 and Rᵢ is
   (i) cycloalkyl of 3 to 10 carbon atoms, inclusive;
   (ii) phenyl; or
   (iii) substituted phenyl 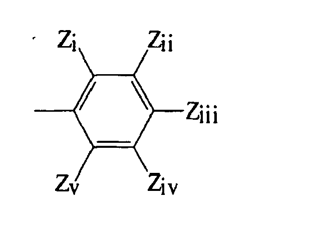 wherein Zᵢ, Z_{iii,}, and Zᵥ are each independently selected from the group consisting of hydrogen, -NO₂, -CN, -C(=O)-R₁, and -SO₃H;
      wherein Zᵢᵢ and Zᵢᵥ are each independently selected from the group consisting of halogen, methyl, hydrogen, methoxy, and hydroxyl;
(c)

   -RₐQₐR_{b}

   wherein Qₐ = -O- or -S-; and
   wherein Rₐ is alkylene of 0 to 6 carbons atoms, inclusive, which may be straight chain or branched;
   wherein R_{b} is either alkyl of 0 to 8 carbon atoms, inclusive, which may be straight chain or branched or substituted phenyl;
(d)

   -C(Rᵢᵢᵢ)(Rᵢᵥ)-Rᵢ

   wherein Rᵢᵢᵢ and Rᵢᵥ are selected independently from the group consisting of
   (i) H; or
   (ii) CHₐZ_{b} where a + b = 3, a = 0 to 3, b = 0 + 3, and
      wherein any Z is selected from the group consisting of halogen; or
(e) haloalkyl of 1 to 8 carbon atoms, inclusive, and 1 to 6 halogen atoms, inclusive, straight chain or branched; but excluding the C-1 position amides, C-1 position alkanoates, and pharmaceutically acceptable C-1 position salts of (5S, 14R, 15S)-trihydroxy-6E, 8Z, 10E, 12E-eicosatetraenoic acid (LXB₄); C-5, C-6, and C-5 position alkanoates of LXB₄.

In another embodiment of this invention, lipoxin analogs have the structural formula VI: 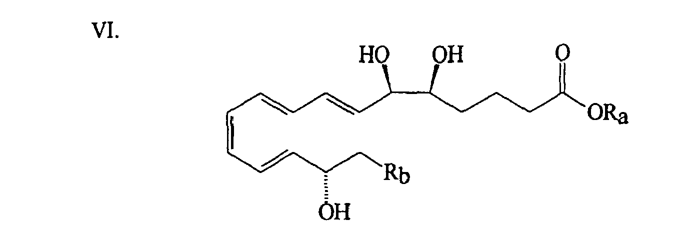 wherein Rₐ is selected from the group
(a) H; or
(b) alkyl of 1 to 8 carbon atoms;
wherein R_{b} selected from the group consisting of:

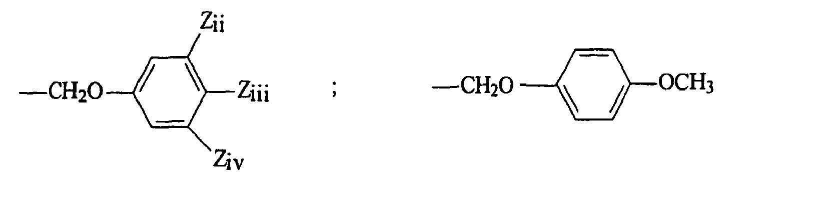

In another preferred embodiment of this invention, lipoxin analogs have the following structural formula VII: 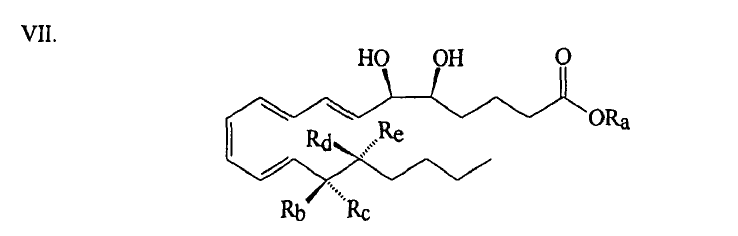 wherein Rₐ is selected from the group
(a) H; or
(b) alkyl of 1 to 8 carbon atoms;
wherein R_{b} and R_{c} are independently selected from the group
(a) H;
(b) hydroxyl, or thiol;
(c) methyl or halomethyls including -CF₃ and -CH₂F;
(d) halogen;
(e) alkoxy of 1 to 3 carbon atoms, including methoxy;
wherein R_{d} and Rₑ are selected independently from the group
(a) H;
(b) hydroxyl, or thiol;
(c) methyl or halomethyl including -CF₃ and -CH₂F;
(d) halogen;
(e) alkoxy of 1 to 3 carbon atoms, inclusive, including methoxy; or
(f) alkyl or haloalkyl of 2 to 4 carbon atoms, inclusive, which may be straight chain or branched; but excluding the C-1 position amides, C-1 position alkanoates, and pharmaceutically acceptable C-1 position salts of (5S, 6R, 15S)-trihydroxy-7E, 9E, 11Z, 13E-eicosatetraenoic acid (LXA₄); C-5, C-6, and C-15 position alkanoates of LXA4.

In another preferred embodiment of this invention, the lipoxin analogs have the structural formula VIII: 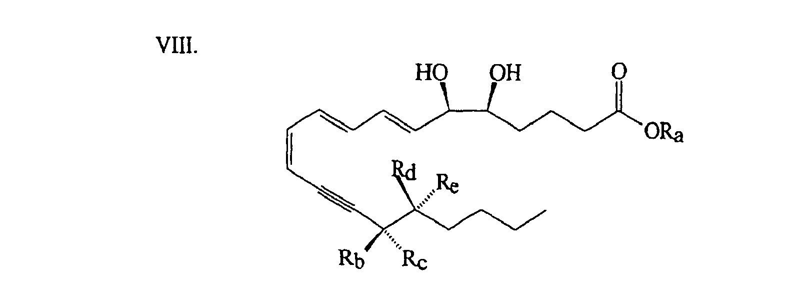 wherein Rₐ is selected from the group
(a) H; or
(b) alkyl of 1 to 8 carbon atoms;
wherein R_{b} and R_{c} are independently selected from the group
(a) H;
(b) hydroxyl or thiol;
(c) halomethyl, including CF_{3:}
(d) halogen;
(e) alkyl of 1 to 3 carbon atoms, inclusive, straight chain or branched; or
(f) alkoxy of 1 to 3 carbon atoms, inclusive;
wherein R_{d} and Rₑ are selected independently from the group
(a) H;
(b) hydroxyl, or thiol;
(c) methyl or halomethyl including -CF₃ and -CH₂F;
(d) halogen;
(e) alkoxy of 1 to 3 carbon atoms, inclusive, including methoxy; or
(f) alkyl or haloalkyl of 2 to 4 carbon atoms, inclusive, which may be straight chain or branched.

In another preferred embodiment of this invention, the lipoxin analogs have the structural formula IX: 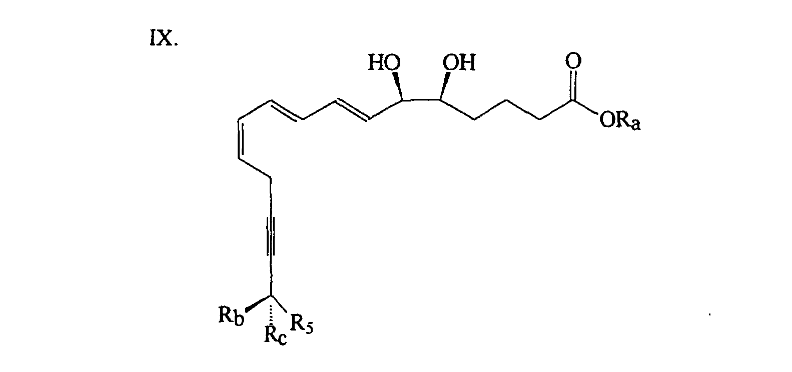 wherein Rₐ is selected from the group
(a) H; or
(b) alkyl of 1 to 8 carbon atoms;
wherein R_{b} and R_{c} are independently selected from the group
(a) H;
(b) hydroxyl or thiol;
(c) halomethyl, including CF₃ and CH₂F;
(d) halogen;
(e) alkyl of 1 to 3 carbon atoms, inclusive, straight chain or branched;
(f) alkoxy of 1 to 3 carbon atoms, inclusive; and
wherein R₅ is
(a) alkyl of 1 to 9 carbon atoms which may be straight chain or branched;
(b)

   -(CH₂)ₙ -Rᵢ

   wherein n = 0 to 4 and Rᵢ is
   (i) cycloalkyl of 3 to 10 carbon atoms, inclusive;
   (ii) phenyl; or
   (iii) substituted phenyl 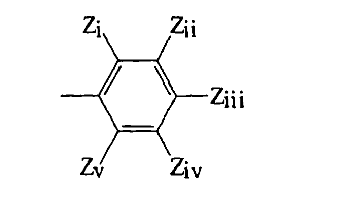 wherein Zᵢ, Z_{iii,}, and Zᵥ are each independently selected from the group consisting of hydrogen, -NO₂, -CN, -C(=O)-R₁, and -SO₃H;
      wherein Zᵢᵢ and Zᵢᵥ are each independently selected from the group consisting of halogen, methyl,
      hydrogen, methoxy, and hydroxyl;
(c)

   -RₐQₐR_{b}

   wherein Qₐ = -O- or -S-;
   wherein Rₐ is alkylene of 0 to 6 carbons atoms, inclusive, which may be straight chain or branched;
   wherein R_{b} is either alkyl of 0 to 8 carbon atoms, inclusive, which may be straight chain or branched or substituted phenyl;
(d)

   -C(Rᵢᵢᵢ)(Rᵢᵥ)-Rᵢ

   wherein Rᵢᵢᵢ and Rᵢᵥ are selected independently from the group consisting of
   (i) H; or
   (ii) CHₐZ_{b} where a + b = 3, a = 0 to 3, b = 0 + 3
      wherein any Z is selected from the group consisting of halogen; or
(e) haloalkyl of 1 to 8 carbon atoms, inclusive, and 1 to 6 halogen atoms, inclusive, straight chain or branched.

In another preferred embodiment, the compounds have the structural formula X: 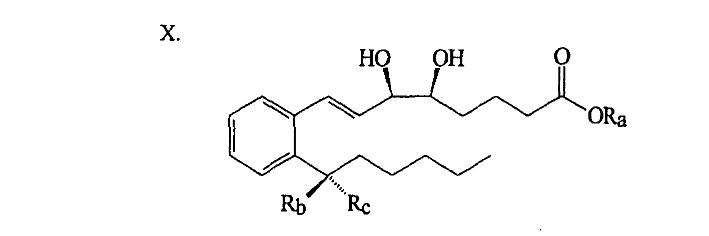 wherein Rₐ is selected from the group
(a) H; or
(b) alkyl of 1 to 8 carbon atoms, inclusive, straight chain or branched; and
wherein R_{b} and R_{c} are independently selected from the group
(a) H;
(b) hydroxyl or thiol;
(c) halomethyl, including, for example, CF_{3;}
(d) halogen;
(e) alkyl of 1 to 3 carbon atoms, inclusive, straight chain or branched;
(f) alkoxy of 1 to 3 carbon atoms, inclusive, including methoxy.

In another preferred embodiment, the compounds have the structural formula XI: 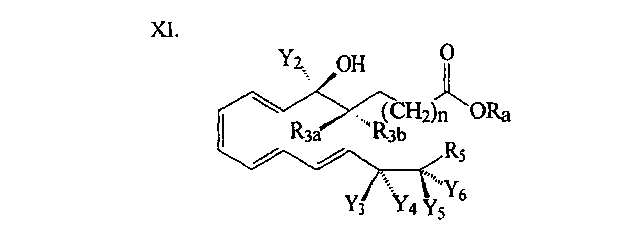 wherein Rₐ is
(i) hydrogen;
(ii) alkyl of 1 to 8 carbons atoms, inclusive, which may be straight chain or branched; or
(iii) detectable label molecule;
wherein n = 1 to 10, inclusive;
wherein Y₂, R₃ₐ, and R_{3b} are independently selected from
(a) H;
(b) alkyl of 1 to 8 carbon atoms, inclusive, which may be straight chain or branched;
(c) cycloalkyl of 3 to 6 carbon atoms, inclusive;
(d) alkenyl of 2 to 8 carbon atoms, inclusive, which may be straight chain or branched; or
(e) RₐQ₂R_{b}
   wherein Q₂ is -0- or -S-;
   wherein Rₐ is alkylene of 0 to 6 carbons atoms, inclusive, which may be straight chain or branched; and wherein R_{b} is alkyl of 0 to 8 carbon atoms, inclusive, which may be straight chain or branched;
wherein Y₁ is -OH , methyl, or -SH ;
wherein Y₂ is
(a) H;
(b) CHₐZ_{b}
   where a + b =3, a= 0 to 3, b = 0 to 3
   Z is halogen; or
(c) alkyl of 2 to 4 carbon atoms, inclusive, straight chain or branched;
wherein Y₃ and Y₅ are independently selected from the group consisting of :
(a) H;
(b) CHₐZ_{b}
   wherein a + b =3, a= 0 to 3, b = 0 to 3 and any Z is cyano, nitro, or halogen; or
(c) alkyl of 2 to 4 carbon atoms, inclusive, straight chain or branched;
wherein Y₄ and Y₆ are independently selected from the group consisting of:
(a) H;
(b) alkyl of 2 to 4 carbon atoms, inclusive, straight chain or branched;
(c) alkoxy of 1 to 4 carbon atoms, inclusive, straight chain or branched; or
(d) hydroxyl or thiol; and
wherein R₅ is
(a) alkyl of 1 to 9 carbon atoms which may be straight chain or branched;
(b)

   -(CH₂)ₙ -Rᵢ

   wherein n = 0 to 3 and Rᵢ is
   (i) cycloalkyl of 3 to 10 carbon atoms, inclusive;
   (ii) phenyl;
   (iii) substituted phenyl 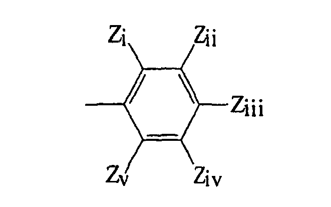 wherein Zᵢᵢ and Zᵢᵥ are each independently selected from the group consisting of halogen, methyl, hydrogen, methoxy, and hydroxyl;
(c)

   -RₐQₐR_{b}

   wherein Qₐ = -O- or -S-;
   wherein Rₐ is alkylene of 0 to 6 carbons atoms, inclusive, which may be straight chain or branched;
   wherein R_{b} is 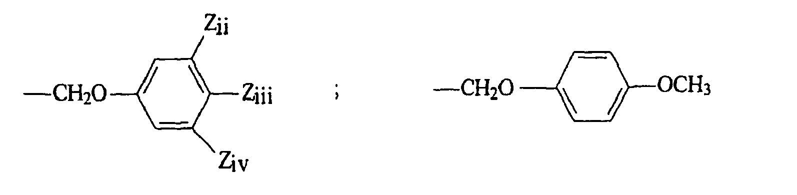
(d) haloalkyl of 1 to 8 carbon atoms, inclusive, and 1 to 6 halogen atoms, inclusive, straight chain or branched;or but excluding the C-1 position amides, C-1 position alkanoates, and pharmaceutically acceptable C-1 position salts of (5S, 14R, 15S)-trihydroxy-6E, 8Z, 10E, 12E-eicosatetraenoic acid (LXB₄); C-5, C-6, and C-5 position alkanoates (acetates) of LXB₄.

In the most preferred embodiment of this invention, the compounds of this invention have the following structural formulas: 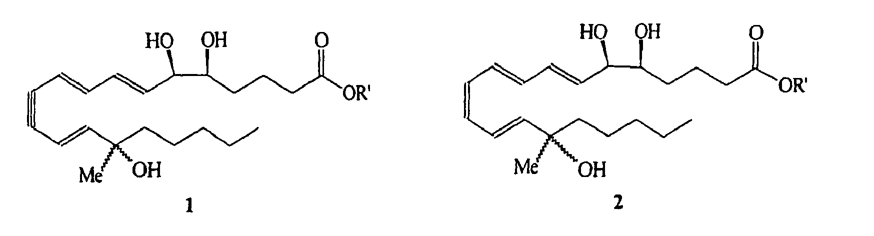 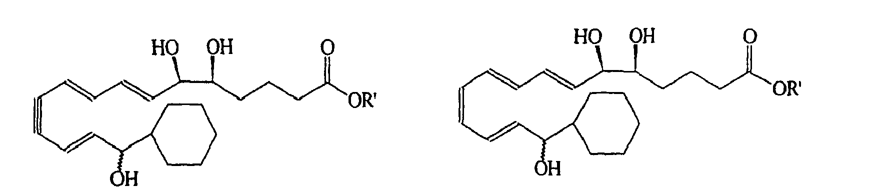 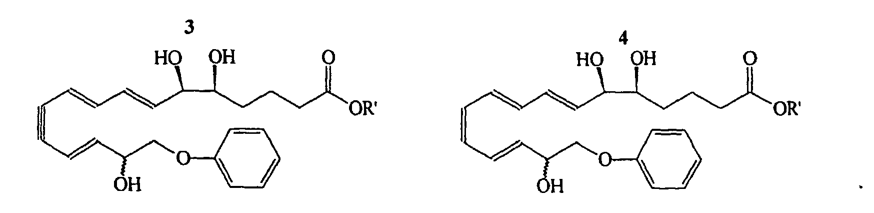 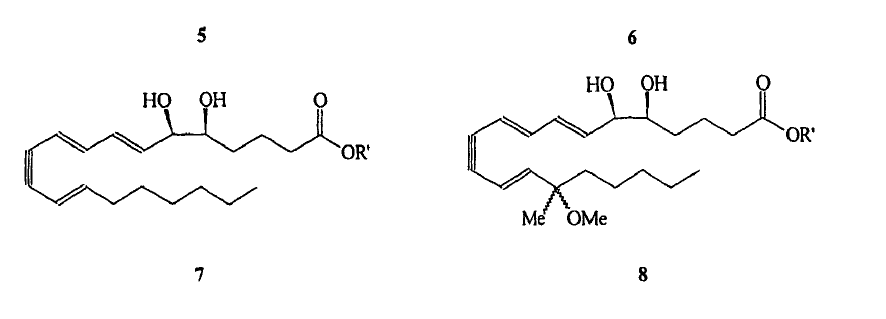 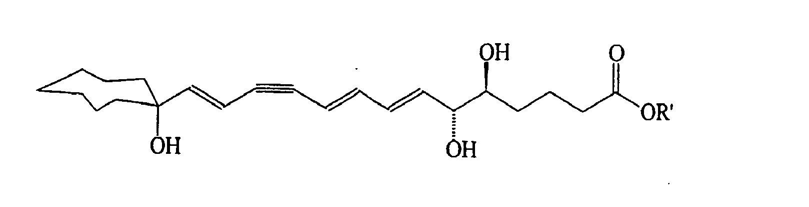 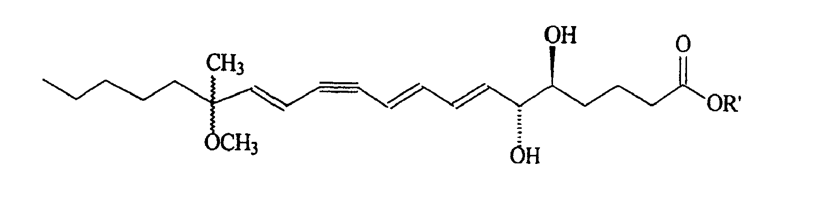 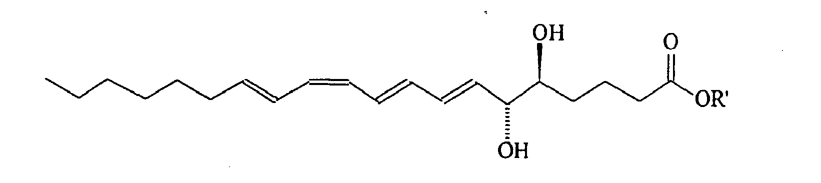 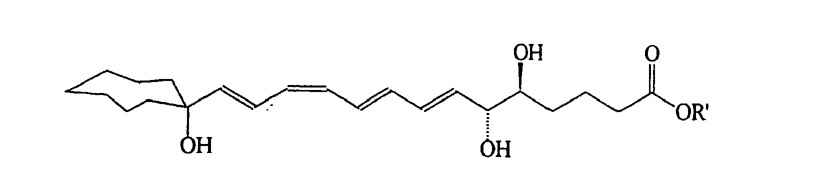 where R' is H or CH₃.

In other preferred embodiments of this invention, the compounds of this invention have the following structural formulas:

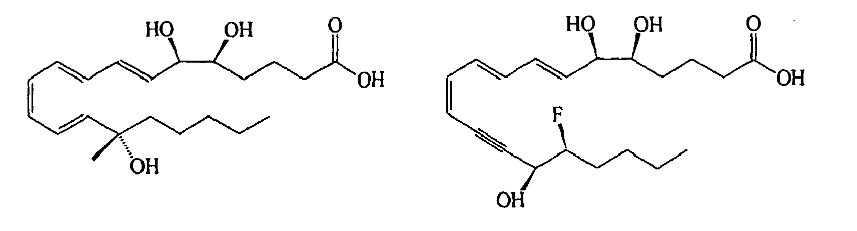

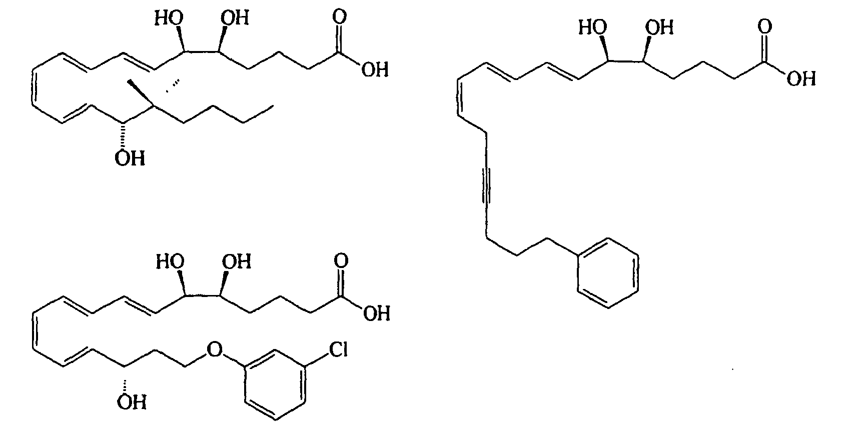

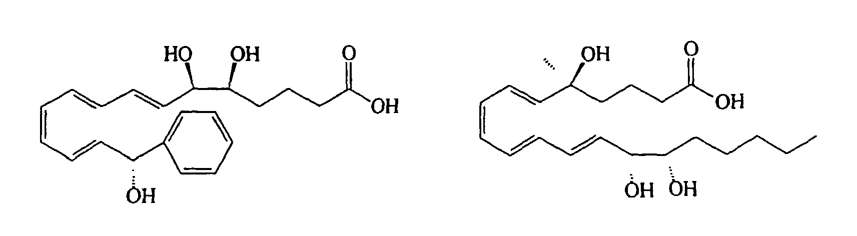

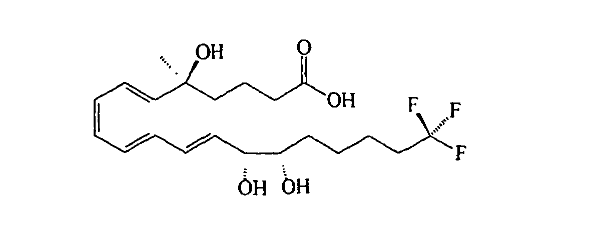

### Method for Making Lipoxin Compounds

Preferred compounds can be made as specifically described in the following Example 1. Other compounds of this invention can be made by combined strategies for lipoxin (LX) and prostaglandin analog synthesis using standard chemical methods such as selective hydrogenation, Pd(0)-Cu(I) coupling, Wittig-type coupling, Sharpless epoxidation, and asymmetric reductions following coupling of the major intermediates described below and in the literature to generate the stable LX analogs of this invention.(Webber, S.E. et al. (1988) Adv. Exp. Med. Biol. 229:61; Radüchel, B. and Vorbrüggen, H. (1985) Adv. Prostaglandin Thromboxane Leukotriene Res. 14:263; and Nicolaou, K.C. et al. (1991) Angew Chem. Int. Ed. Engl. 30:1100). Geometrical variations can be accomplished e.g. as described in U.S. Patent No. 4,576,758 and Nicolaou, K.C. (1989) J. Org. Chem. 54: 5527.

As shown below, lipoxin analog compounds comprising subgenus 1 in Scheme I can be prepared as three major fragments (A, B, and C), which can then be combined to form the total molecule.

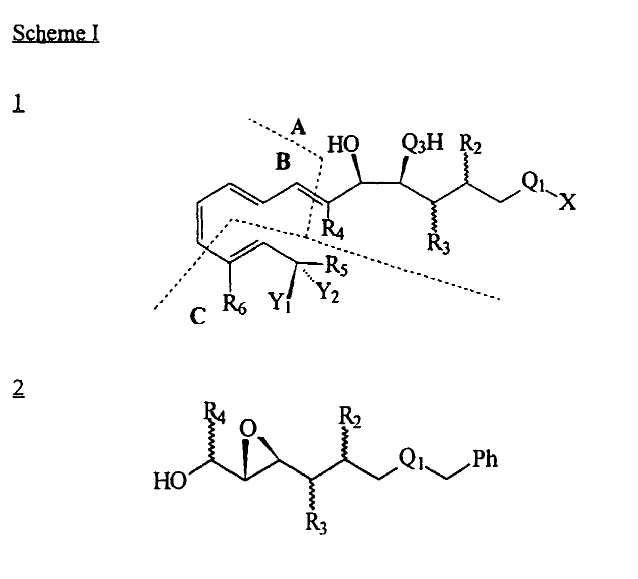

Synthesis of the epoxy-alcohol for precursor fragment 2 can be generated with substitutents R₂, R₃ and R₄ selected from hydrogen, phenyl, halogen or methyl. Each of these respective epoxy-alcohols may be transformed into phenyl urethane derivatives as 3. 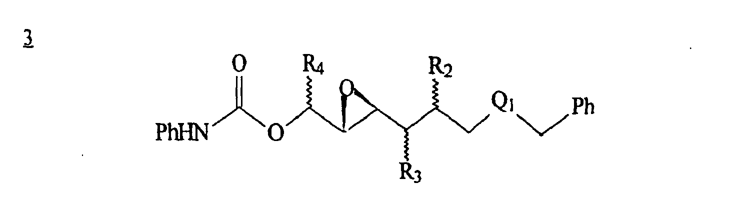 with PhNCO, pyrimidine and CH₂Cl₂ followed by Lewis acid catalysis by SN² opening to give a 1,2 cyclic carbonate that contains the vicinal diol at C-6 in the (R) configuration required for binding and C-5 in the (S) configuration also established for bioactivity and binding at a recognition site. These alcohols are next protected to generate the precursor A fragment as 4.

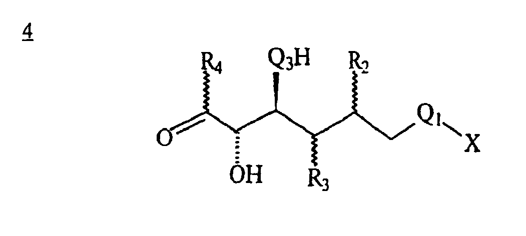

These A fragments can now be coupled to the fragment B intermediate, a phosphonium bromide 5 as in Webber, S.E. et al. (1988) Adv. Exp. Med. Biol. 229:61 in gram quantities to generate the combined A + B fragment products 6.

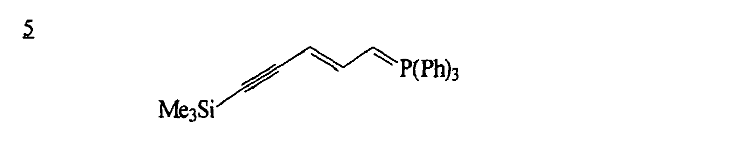

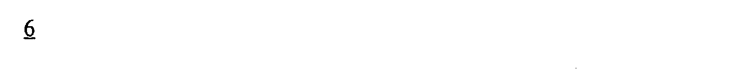

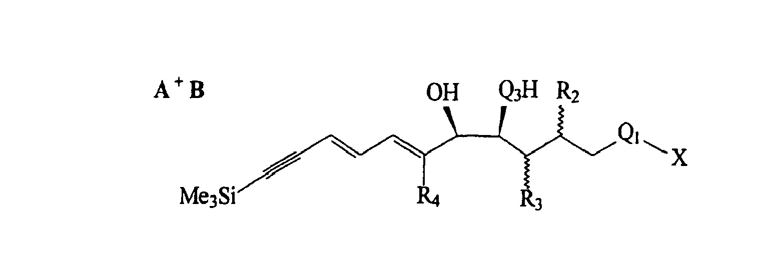

Fragment C intermediates from Scheme I are generated in parallel to preparation of A-B couplings. In these C fragments, substitutions at Y₁ and/or Y₂ are methyl, methoxy, hydrogen, cyano, nitro, or halogen; see specific example 3. Thus, carrying 15-methyl and/or, for example, 16-methyl or 16-phenoxy-derivatives permits these substituted-LXA₄ analogs to be not susceptible to dehydrogenation.

Thus, the C fragments carrying the preferred resistance to enzymatic oxidation and/or dehydrogenation may be converted by protection of key sites, followed by bromination to give vinyl bromide products of fragment C such as 6b that is coupled to 6 by using catalytic amounts of P(Ph₃)₄ and CuI to generate the complete backbone structure of the LXA₄ analogs of genus formula I. This scheme is further illustrated by the following Examples.

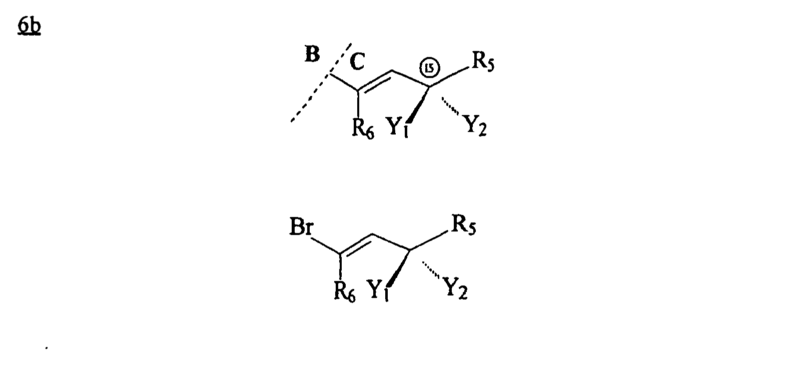

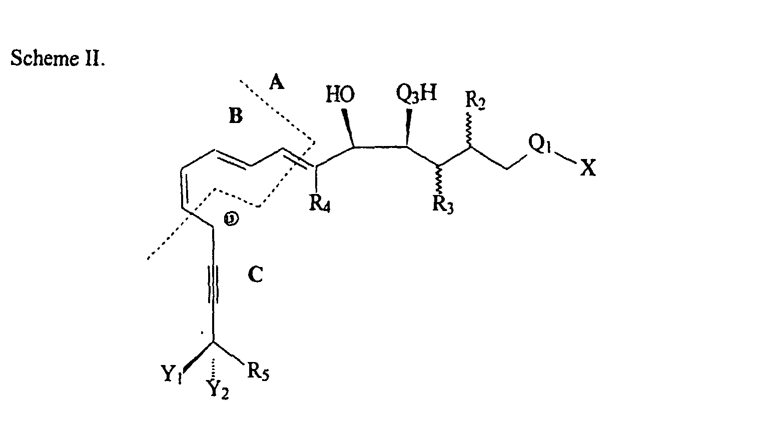

The compounds of this invention within subgenus formulas II and III may be made synthesized in a similar manner.

Compounds in genuses II and III are generated by first individually preparing substituted compounds of fragment A that are each coupled as in Scheme I to individually prepared fragment B to generate 7 or A₁ + B₁ fragments possessing individual substitutions at X, Q₁, R₂, R₃, and R₄ as indicated.

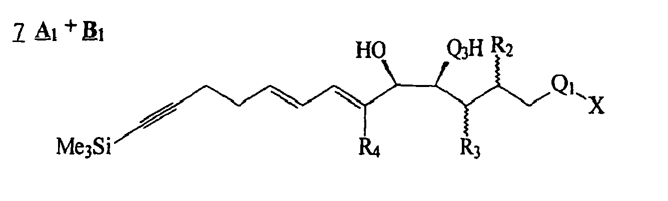

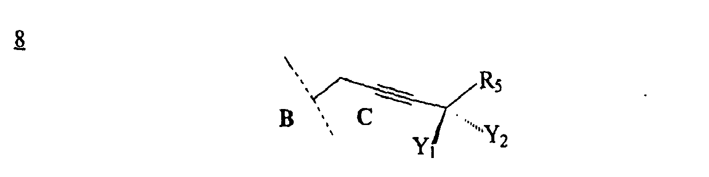

The C₁ fragment 8 carrying the acetylenic group C-14,15 and the ω-C-20 end substitutions will each be generated as shown above for structure 6 prostaglandin analogs and converted to their corresponding vinyl bromide products as in (KCN JAC 1985, Webber) to yield brominated products of each individual substituted fragment C₁ or 8 species that are suitable for coupling to 20 using catalytic amounts of P(Ph₃)₄ and CuI to generate the combined products of the acetylenic-LXA₄ analog class. Each of the final products may then be subject to gradient RP-HPLC using rapid diode array detection (as in Serhan, C.N., Methods in Enzymology) for purification. The presence of the modification at C-15 thru C-20 of LXA₄ can alter metabolism by dehydrogenases and oxidases by providing steric hindrance, stable prostaglandin analogs carrying C-15 to ω-end substitutions have been prepared and are not metabolized by dehydrogenases (Radüchel, B. and Vorbrüggen, H. (1985) Adv. Prostaglandin Thromboxane Leukotriene Res. 14:263 and Vorbrüggen, H. et al. In: Chemistry, Biochemistry, and Pharmacological Activity of Prostanoids (Roberts, S.M., Scheinmann, F. eds.). Oxford: Pergamon Press).

The cyclo-LXA₄ compounds of this invention within genus formula IV may be made in the following manner.

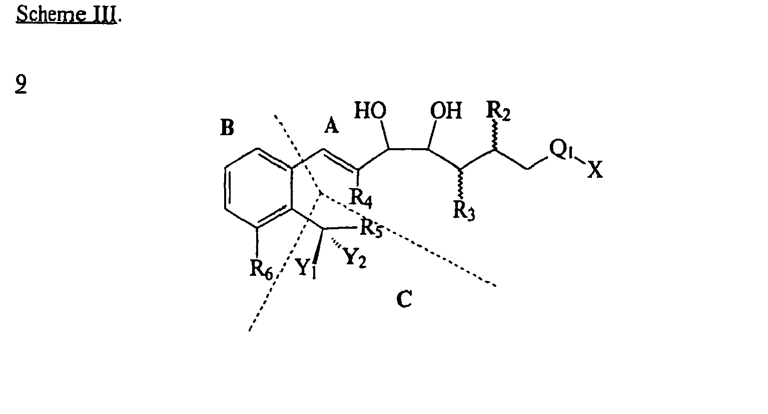

The parent compound of this class is also subject to a similar total synthesis strategy and is assigned three main fragments A, B, and C in structure 30. A precursor for fragment A may be prepared by routes used in Nicolaou, K.C. (1989) J. Org. Chem. 54:5527 to prepare 10 in the synthesis of 7-*cis*,11-*trans*-LXA₄ methyl ester.

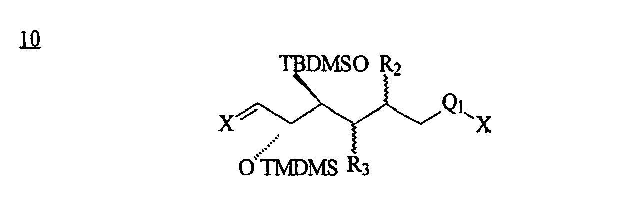

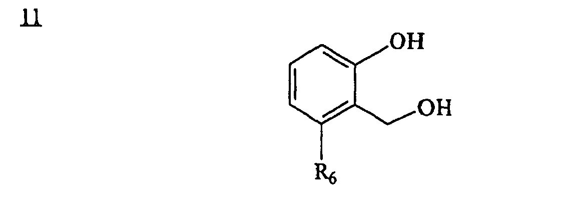

Fragment B in 30 can be obtained via the precursor 11 or saligenin-([*O*-hydroxybenzylalcohol) as generated in (Vorbrüggen et al., p. 353). The benzyl alcohol 11 is reacted with 10 (1:1) in the presence of NaH in DMF to give 12. This key intermediate is silylated in BSTFA followed by coupling with individual fragments designed for C precursors.

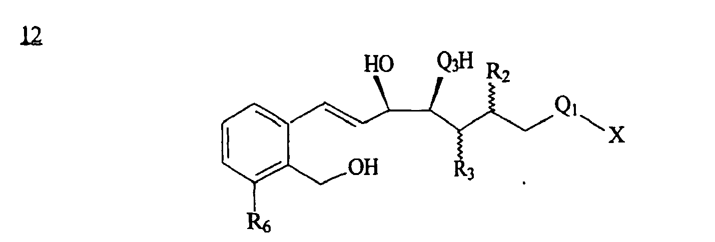

13 can then be coupled to vinyl brominate fragment C of given individual design by treating the bromite precursor with 4.0 equivalents of AgNO₃, then 7.0 equiv. of KCN, EtOH/THF/H₂O (1:1:1), 0 ⇒ 25°C, 2-4 h. the individual products are then subject to Lindlar cat. for selective catalytic mild hydrogenation in CH₂Cl₂ 2-3 h to give individual compounds belonging to the genus IV. Each can be saponified in LiOH/THF to give corresponding free acids after isolation by RP-HPLC.

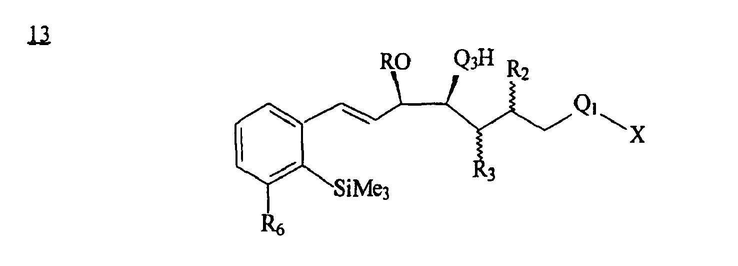

The invention of genus IV compounds is illustrated further by example 5 below, the synthesis of 15(±)methyl-cyclo-LXA₄ methyl ester and corresponding free acid.

The compounds of this invention within genus formula V may be made in the following manner. Several systems studied with LXB₄ indicate that several sites within the natural compound are required for bioactivity (Serhan, C.N. (1991) J. Bioenerg. and Biomembr. 23:105). These sites include the C-14 alcohol in the (R) configuration and the double bond at C-8,9 of the tetraene in the *cis* configuration. In addition, based on metabolic studies resulting in the instant invention, several key addition sites have been identified as being necessary to preserve LXB₄ bioactivity. These include preserving the C-15 alcohol from dehydrogenase activity (i.e., block 5-oxo-LXB₄ formation); maintaining both the Δ8 bond and 14(R) alcohol; and preventing reduction of Δ6-7 double bond and β/ω -oxidations of the resultant compounds.

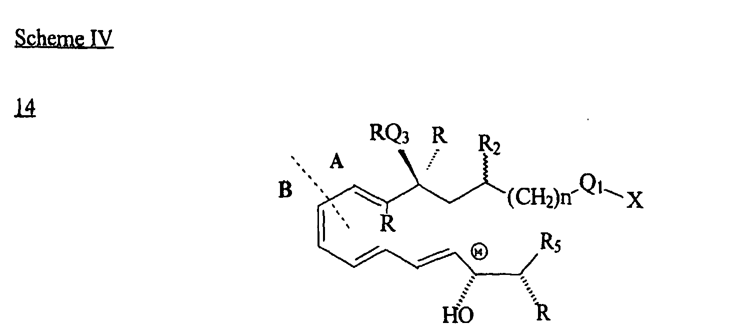

Thus, genus V (14) maintains the regions of LXB₄ which are necessary for its bioactivity, but modifies the regions available to metabolic degradation. Again, a retrosynthetic analysis gives priority to three key fragments A, B and C designated in 14 Coupling of key intermediates to generate members of the LXB₄ analog class uses standard techniques as outlined for LXA₄ and its analogs namely, selective hydrogenation (to generate 8-*cis* geometry); Pd(0)-Cn(I) coupling to join A & B fragments carrying unique substitutions; Wittig-type coupling to join C fragments that carry the required substitutions and Sharpless epoxidation to yield the 14(R) vicinal alcohol (see ref. Nicolaou, K.C. et al. (1991) Angew. Chem. Int. Ed. Engl. 30:1100.), ref.; Weber, S.E. et al. (1988) Adv. Exp. Med. Biol. 229:61 Ed. Wong, P.K. and Serhan, C.N.) and those cited within). Thus by using a similar strategy to LXA₄ analogs and the construction of native LXB₄ the specific analogs can be obtained.

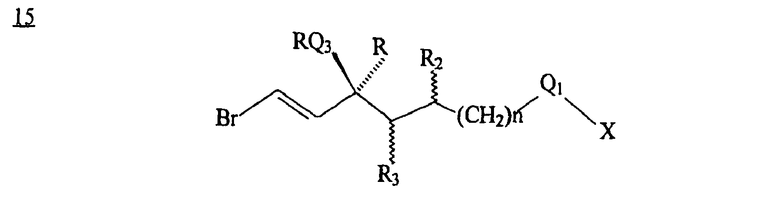

The A fragments of 15 that carry substitutions at R₂, R₆, R₇ that can be (H, CH₃, OCH₃, phenyl, halo-substituted phenyl are generated by standard methods from, for example 16 where= CH₂ of increasing chain length. 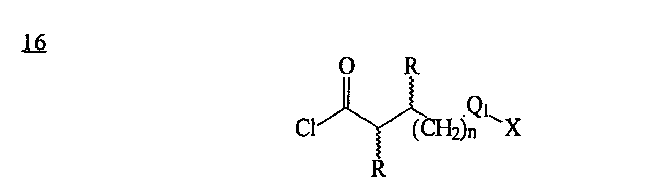 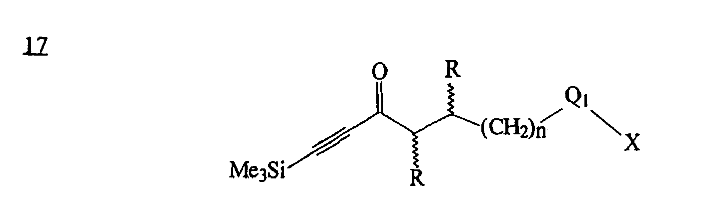 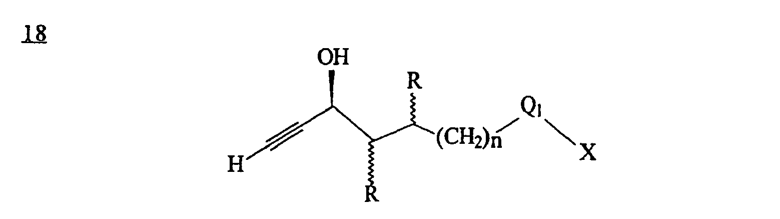 Compound 16 is converted to the vinyl bromide 15 as in (Nicolaou K.C. et al. (1991) Angew. Chem. Int. Ed. Engl. 30: 1100-16.) via a trimethylsilyl acetylenic intermediate 17 that is reduced by pinanyl-9BBN then n- BuN₄NF in THF to yield 18 than is now submitted for bromination after protecting essential moieties such the alcohol to give 15 (fragment A).

The fragment C in 14 is generated from compound 19 with the R₅ substitutions as indicated.

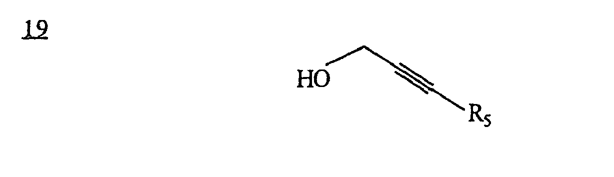

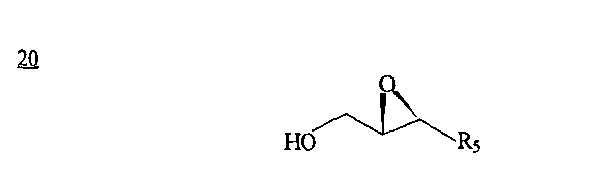

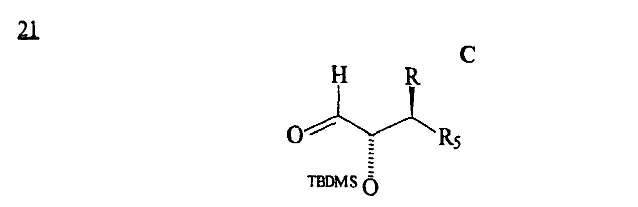

The acetylenic alcohol 19 is then reduced in LAH followed by Sharpless asymmetric epoxidation to generate 20 that is isolated by RP-HPLC to yield the (+) isomer that is used to generate the required C-14 alcohol of LXB₄ analogs in the (R) configuration compound 20 is transformed to the corresponding aldehyde 21 after protection of the substituted groups carried at R₄ and R₅ as well as the alcohol using PCC in methylene chloride. (Nicolaou, K.C. et al. (1991) Angew. Chem. Int. Ed. Engl. 30:1100).

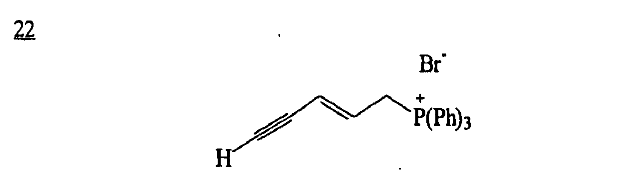

The phosphonium salt 22 can be prepared as in Ref.; (Weber, S.E. et al. (1988) Adv. Exp. Med. Biol. 229:61, Ed. Wong, P.K. and Serhan, C.N.) and used here to generate the B-C fragment coupling via a Wittig-type coupling to give 23 carrying the designated substitutions in the group of 23
where R₄ and R₅ carry substitutions. This *cis* double bond of 23 can be is isomerized to give the trans isomer using I₂ as a catalyst to give the parent precursor form of 14 as 24.

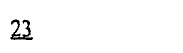

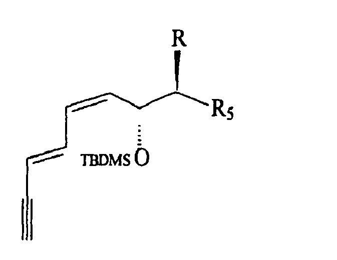

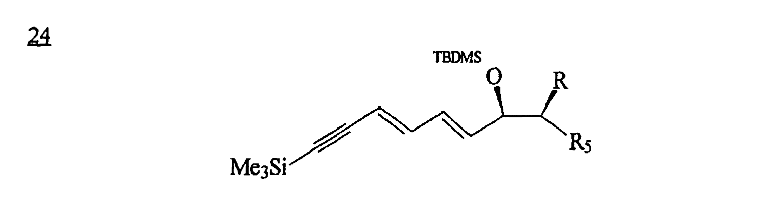

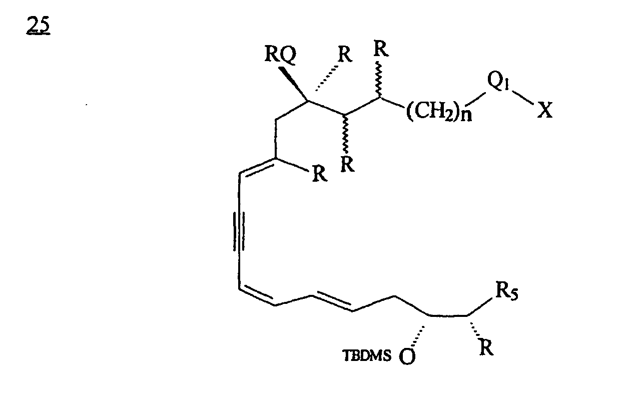

Then coupling of 24 to 15 is accomplished by Pd(O) - Cu(I) coupling to give the acetylenic precursor of 14 designated 25. Following selective Lindlar catalytic hydrogenation the individual LXB₄ analogs can be further purified via RP-HPLC used the tetraene skeleton as a convenient means to isolate individual products employing rapid diode array detection (Serhan, C.N. (1990) Meth. Enzymol. 187:167).

### Utilities

The compounds of this invention have the biological activity of natural lipoxins, but are more resistant to degradation or alternatively inhibit the degradation of natural lipoxins. The disclosed compounds therefore have utility as pharamaceticals for treating or preventing a number of diseases or conditions associated with inadequate or inappropriate lipoxin mediated cellular response in a subject.

Based on the cell stimulatory action of lipoxins, the invention provides a method for treating a subject with a myeloid suppressive disorder by administering to the subject an effective amount of a pharmaceutical composition comprising a lipoxin analog. The effective amount is ordinarily that amount which is required to assure sufficient exposure to the target cell population. Such an amount will ordinarily depend upon the nature of the analog, the mode of administration, the severity of the myeloid suppression, and other factors considered by a person of ordinary skill when determining a dosage regimen.

Therapeutic use of a cell proliferative lipoxin analog also includes removing cells from a subject, stimulating cell growth in vitro, and reintroducing the enhanced cell preparation, in whole or in part, into the subject. Additional therapeutic agents (e.g. cytokines such as GM-CSF) may be optionally used in conjunction with the lipoxin during stimulation or in conjunction with the introduction of the cell preparation.

In another embodiment, the compounds of this invention are used to treat or prevent inflammation or an inflammatory response. LXA₄ inhibits the activation of leukocytes which are mediators of inflammation. The LXA₄ -induced effect includes inhibition of leukocyte migration, generation of reactive oxygen species, and the formation of pro-inflammatory mediators involved in tissue swelling. (Raud, J. et al. (1991) Adv. Exp. Med. Biol. 314:185. Cell-Cell Interactions in the Release of Inflammation Mediators vol. 314) LXB₄ exhibits radioprotective actions, such as preventing diarrhea and ataxia, in an *in vivo* assay with mouse hematopoietic stem cells. (Walken, T.L. Jr.,(1988) J. Radiat. Res. 29:255)

The leukocyte-mediated inflammation or inflammatory responses cause or contribute to a wide variety of diseases and conditions including various forms of asthma and arthritis. Included within the present invention are inflammatory responses to physical injury, such as physical trauma, radiation exposure, and otherwise.

In another embodiment, the compounds of this invention are used to treat or prevent inflammation by antagonizing the action of leukotrienes. LXA₄ inhibits LTB₄-induced inflammation, blocking both plasma leakage and leukocyte migration in an *in vivo* assay of the hamster cheek pouch. (Hedqvist, P. et al. (1989) Acta Physiol. Scand. 137 : 571. ) Plasma leakage and leukocyte migration are key events in both wound healing and inflammation. LXA₄ also antagonizes LTD₄-induced renal hemodynamic actions and blocks the binding of LTD₄ to mesangial cells which are responsible, in part, for regulating hemodynamics in the kidney. (Badr. K.F. et al. (1989) Proc. Natl. Acad. Sci. USA 86: 438.)

The compounds of this invention may be administered to antagonize the action of sulfidopeptide leukotrienes, such as LTD₄, LTC₄, and LTB₄. Leukotriene-mediated vasoconstrictive responses are associated with diseases such as: asthma, anaphylactic reactions, allergic reactions, shock, inflammation, rheumatoid arthritis, gout, psoriasis, allergic rhinitis, adult respiratory distress syndrome, Crohn's disease, endotoxin shock, traumatic shock, hemmorrhagic shock, bowl ischemic shock, renal glomerular disease, benign prostatic hypertrophy, inflammatory bowl disease, myocardial ischemia, myocardial infarction, circulatory shock, brain injury, systemic lupus erythematosus, chronic renal disease, cardiovascular disease, and hypertension.

In another embodiment, the compounds of this invention are used to treat or prevent a vasocontractive response or condition. Lipoxins induce endothelium-dependent vasodilation (LXA₄) (Lefer, A.M. et al (1988) Proc. Natl. Acad. Sci. USA 85:8340) and dilation of cerebral arterioles in new born pigs *in vivo* (LXA₄ and LXB₄) (Busija, D.W. et al. (1989) Am. J. Physiol. 256:468.). Furthermore, LXA₄ induces rapid arteriolar dilation in hamster cheek pouch *in vivo* (Dahlen, S.-E. et al. (1987) Acta Physiol. Scand. 130:643 ) and in the renal hemodynamics of the rat. ( Badr, K.F. et al. (1987) Biochem. Biophys. Res. Commun. 145: 408).

Vasocontractive responses or conditions cause, contribute, or are associated with diseases and conditions such as renal hemodynamic diseases, including glomerular diseases, cardiovascular diseases including hypertension, myocardial infarction, myocardial ischemia, and vascular diseases and gastrointestinal diseases.

Also encompassed by this invention is a method of screening lipoxin analogs or other compounds to identify those having a longer tissue half-life than the corresponding natural lipoxin. This method can be used to determine whether the compound inhibits, resists, or more slowly undergoes metabolism compared to the natural lipoxin. This method is performed by preparing at least one enzyme which metabolizes lipoxins, contacting the compound with the enzyme preparation, and determining whether the compound inhibits, resists, or more slowly undergoes metabolism by the enzyme. Cells having a lipoxin recognition site, such as polymorphonuclear neutrophils, peripheral blood monocytes, and differentiated HL-60 cells are among the appropriate sources for the enzyme preparation. The lipoxin recognition site may exist naturally, or be induced artificially, by a disease state, or by an injury. A non-limiting example of artificially-induced LXA₄ recognition sites is the induction of such sites in differentiated HL-60 cells.

In one embodiment, preparation of the enzymes comprised harvesting cells and performing freeze-thaw lysis three times, followed by ultracentrifugation to yield a 100,000g supernatant. A cell-free 100,000g pellet may also be used. In addition, an enzyme preparation may comprise any enzymes that do not participate in natural lipoxin metabolism, but perform transformations upon lipoxins similar or equivalent to those transformations performed by the enzyme or enzymes which naturally metabolize lipoxins. Nonlimiting examples of appropriate enzymes are 15-hydroxyprostaglandin dehydrogenase, cytochrome P-450 monogenases from human leukocytes, and rat and human liver microsomes.

Characterization of lipoxin metabolites included standard techniques such as extraction, chromatography, and quantitative HPLC followed by trimethyl silyl derivatization, O-methoxime derivatization and gas chromatography/mass spectroscopy analysis. The experimental details of this embodiment are described below in Example 1.

Lipoxin analogs can also be screened for binding activity with a lipoxin receptor recognition site, for example by contacting the compound with a receptor recognition site and determining whether and to what degree the compound binds. Examples of kinetic binding assays include homologous displacement, competitive binding, isotherm , and equilibrium binding assays.

The receptor recognition site may normally exist or it may be induced by a disease state, by an injury, or by artificial means. For example, retinoic acid, PMA, or DMSO may be used to induce differentiation in HL-60 cells. Differentiated HL-60 cells express LXA₄-specific receptor recognition sites. Examples of other cells which may be screened for lipoxin specificity include PMN, epithelial cells, and peripheral blood monocytes.

Selection of competitive ligands will depend upon the nature of the recognition site, the structure of the natural substrate, any structural or functional analogs of the natural substrate known in the art, and other factors considered by a skilled artisan in making such a determination. Such ligands also include known receptor antagonists. The compounds of this invention may be radiolabelled with isotopes including ²H, ³H, ¹³C, and ¹⁴C by standard techniques known in the art of radiochemistry or,synthetic chemistry.

In one embodiment of this method, the structural specificity of induced LXA₄ recognition sites was assessed with LXB₄, LTC₄, LTB₄ and trihydroxyheptanoic methyl ester. The experimental details of this embodiment are described below in Example 2.

In addition, the compounds of this invention may be used to exert certain actions on specific cell types as developmental models for inflammation and injury. For example, LXA₄ stimulates the mobilization of intra-cellular Ca²⁺, lipid remodeling, and chemotaxis without aggregation in human PMN (Palmblad, J. et al. Biochem. Biophys. Res. Commun. (1987) 145: 168; Lee, T.H. et al. Clin.Sci. (1989) 77:195; Nigam, S. et al. J.Cell. Physiol. (1990) 143:512; Luscinskas, F.W. et al. ( 1990) Biochem. Pharmacol. 39:355). LXA₄ also blocks both LTB₄ and FMLP-induced responses, such as IP₃ generation. LXB₄ also stimulates lipid remodeling. LXA₄ activates isolated PKC, and is specific for the γ-subspecies of PKC which is found in the brain and spinal cord. (Hansson, A. et al. Biochem. Biophys. Res. Commun. (1986) 134: 1215; Shearman, M.S. et al. FEBS Lett. (1989) 245: 167); The publication Nicolaou, K.C. et al. Angew. Chem. Int. Ed. Engl. (1991) 30 : 1100 and references cited within are expressly incorporated here by reference.

The present invention is further illustrated by the following examples which should in no way be construed as being further limiting. The contents of all references and issued patents cited throughout all portions of this application including the background are expressly incorporated by reference.

### Examples

### Example 1 Synthesis of Lipoxin Analog Compounds

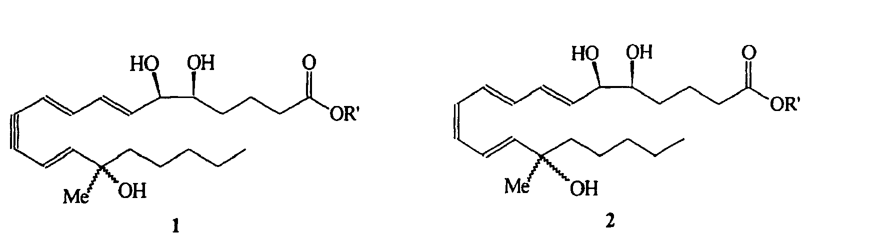

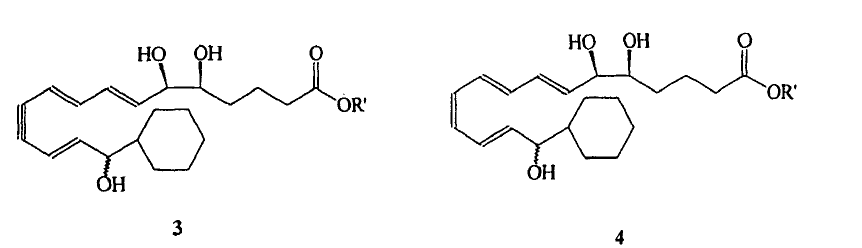

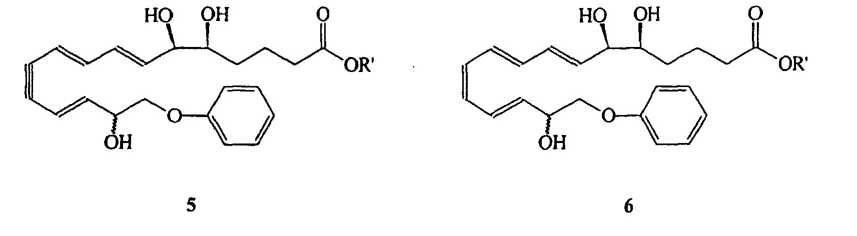

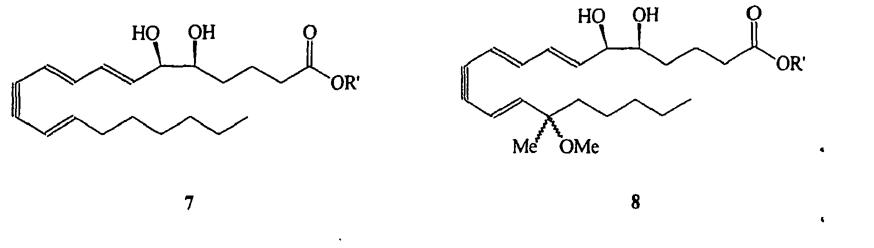

### Preparation of the methyl ester precursor of compound 1;

To a solution of 3-methyl-3-trimethylsiloxy-1-bromo-1-octene (130 mg. 0.44 mmol) in benzene (1.5 mL) was added n-propylamine (0.05 mL, 0.61 mmol) and Pd(PPh₃)₄ (20 mg. 0.02 mmol) and the solution was protected from light. It was then degassed by the freeze-thaw method and stirred at rt for 45 min. (7E, 9E, 5S, 6R) Methyl 5,6-di(tert-butyldimethylsiloxy)-dodeca-7,9-diene-11-ynoate (183 mg. 0.44 mmol) (compound 12) and copper iodide (14 mg. 0.07 mmol) were added and the solution was one more time degassed by the freeze-thaw method. The mixture was stirred for 3 h at rt and quenched with saturated aqueous solution of NH₄Cl and extracted with ether. It was then washed with brine and dried over MgSO₄ and the solvent was evaporated. Flash column chromatography (silica, 3% ether hexanes) afforded pure compound as a colorless liquid (171 mg. 57% yield).

To a solution of the compound (171 mg. 0.25 mmol) in THF (0.5 mL) was added n-BuN₄F(0.9 mL. 0.90 mmol) and the mixture was stirred at rt. The reaction was completed in 2 h at which time it was poured into water and extracted with ether. The ether extracts were washed with brine, dried over Na₂SO₄ and the solvent was evaporated. Flash column chromatography (silica 4% MeOH/CH₂Cl₂) afforded the methyl ester (24 mg.) together with some of the corresponding lactone. HPLC retention time: 9:39 min (microsorb reverse phase, 4.6mm X 25 cm, C-18 column, MeOH/H₂O 70:30 flow rate 1 ml/ min, UV detector at 300nm). UV in MeOH: λₘₐₓ283, 294, 311 nm. ¹H NMR (500 MHz CDCl₃) δ6.53 (dd. 15.2 10.9 Hz, 1 H), 6.32 (dd, J = 15.1, 11.0 Hz, 1 H), 6.17 (d, J=15.9 Hz, 1 H) 5.83 (dd. J = 17.5, 2.1 Hz, 1 H), 5.80 (dd. J = 15.2, 6.7 Hz, 1 H), 5.72 (dd. J = 17.0, 2.1 Hz, 1 H), 4.14 (m, 1 H), 3.68-3.64 (m, 4H), 2.35-2.31 (m, 2 H), 1.51-1.48 (m, 1 H), 1.43-1.42 (m, 2 H), 1.30-1.23 (m, 15 H) 0.85 (t, 3 H). ¹³ C NMR (126 MHz, CDCl₃) δ150.01, 140.18, 132.95, 132.26, 112.43, 107.50, 75.23, 73.76, 42.49, 33.67, 32.17, 31.36, 27.96, 23.56, 22.58, 21.03, 14.03.

### Preparation of the methyl ester precursor of compound 2:

A solution of the methyl ester precursor of compound 1 (3 mg. in CH₂Cl₂ (1 ml) was mixed with Lindlar's catalyst (1 mg.) and placed under a hydrogen atmosphere. The mixture was stirred at rt in the dark followed by HPLC until about 80% conversion (1 h). Filtration over celite evaporation of the solvent and separation by HPLC gave a pure methyl ester. HPLC retention time: 10:02 min (microsorb reverse phase. 10 mm X 25cm C-18 column, MeOH/H₂O 70:30 flow rate 4 ml/min. UV detector at 300nm). UV in MeOH: ηₘₐₓ 287, 301, 315 nm.

### Preparation of the methyl ester precursor of compound 3:

This compound was prepared similarly to the preparation of the methyl ester precursor of compound 1 (from 3-cyclohexyl-3-trimethylsiloxy-1-bromo-1-octene). Desilylation of this compound was also performed in a similar manner to afford the methyl ester. HPLC retention time 8:02 min (microsorb reverse phase, 4.6mm X 25cm. C-18 column, MeOH/H₂O 70:30, flow rate 1 ml/min, UV detector at 300nm). UV in MeOH: λₘₐₓ 282, 293, 311 1 nm. ¹H NMR (360 MHz, CDCl₃) δ6.56 (dd, 15.4, 10.9 Hz, 1 H), 6.33 (dd, J = 15.2, 10.9 Hz, 1 H), 6.13 (dd, J = 15.8, 6.5 Hz, 1 H), 5.81 (dd, J = 15.2, 6.4 Hz, 1 H), 5.80 (d, J = 15.6 Hz, 1 H), 5.73 (dd, J = 15.4,2.1 Hz, 1 H), 4.15 (br, 1 H), 3.93-3.90 (m, 1 H), 3.67 (br, 1 H), 3.65 (s, 3 H), 2.34 (t, 2 H), 1.82-1.65 (m, 10 H), 1.46-1.38 (m, 3 H), 1.26-1.01 (m, 5 H).

### Preparation of the methyl ester precursor of compound 4;

Selective hydrogenation of the methyl ester precursor of compound 3, followed by HPLC purification gave the methyl ester precursor of compound 4. HPLC retention time: 9.72 min (microsorb reverse phase, 10 mm X 25cm C-18 column, MeOH/H₂O 70:30 flow rate 4 ml./min. UV detector at 300nm), UV in MeOH: λₘₐₓ 288, 301, 315 nm. ¹H NMR (250 MHz, C₆D₆) δ 6.66-6.89 (m, 2 H), 5.95-6.24 (m, 4 H), 5.55-5.66 (m, 2 H), 3.82 (m, 1 H), 3.73 (m, 1 H), 3.41 (m, 1 H), 3.31 (s, 3H, OCH₃), 2.08 (t, 2 H, CH₂COO), 1.00-1.81 (m, 18 H).

The methylesters can be converted to corresponding alcohols using standard techniques.

### Synthesis of 15(R)-15-methyl-LXA₄ and 15(±)methyl-LXA₄

Approximately 1 gm acetylenic ketone a is prepared using Friedel-Crafts acylation of bis(trimethylsilyl) acetylene with hexanoyl chloride and is reduced using (-)-pinayl-9-BBN to give the (S) alcohol in CH₃N₂ as in Webber, S.E. et al. (1988) Adv. Exp. Med. Biol. 229:61; Nicolaou, K.C. et al. (1991) Angew. Chem. Int. Ed. Engl. 30:1100; and Vorbrüggen, H. et al.: In: Chemistry, Biochemistry, and Pharmacological Activity of Prostanoids (Roberts, S.M., Scheinmann, F. eds.). Oxford: Pergamon Press, to generate the methyl at C-15.

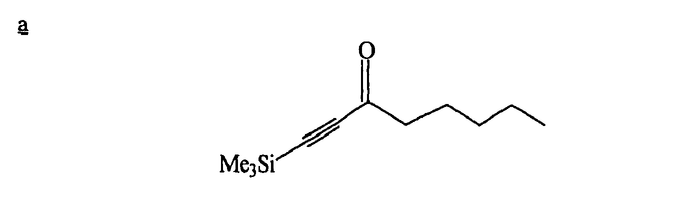

Alternatively, the keto group can be treated with CH₃MgBr (60⇒70°C) as in Vorbrüggen, H. et al.: In: Chemistry, Biochemistry, and Pharmacological Activity of Prostanoids (Roberts, S.M., Scheinmann, F. eds.). Oxford: Pergamon Press to yield the 15(±)methyl of b (2-5 g) in dry CH₂Cl₂ (∼20 ml) at 0°C with sequential additions of 2,6-lutidine (5.2 ml) and tert-butyldimethylsilyl triflate (6.9 ml). This reaction is mixed for 1 h and then diluted with 100 ml ether for aqueous extraction and drying with MgSO₄.

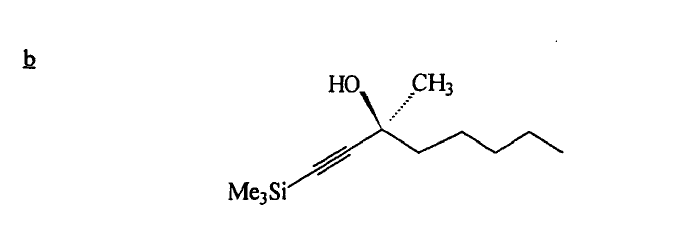

The product c is then coupled with d 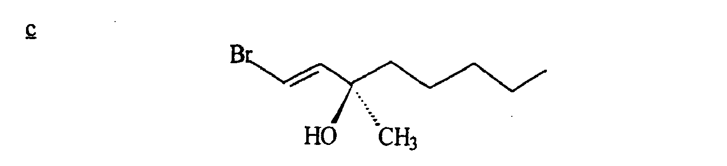 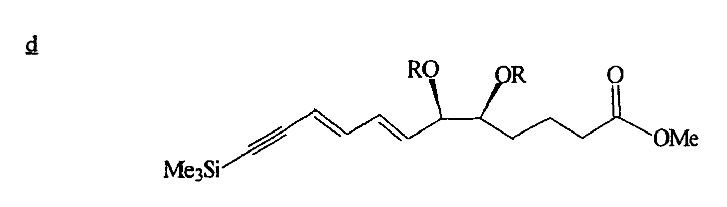 that is generated as in Nicolaou, K.C. et al. (1991) Angew Chem. Int. Ed. Engl. 30:1100; Nicolaou, K.C. et al. (1989) J. Org. Chem. 54:5527 and Webber, S.E. et al. (1988) Adv. Exp. Med. Biol. 229:61. Structure d from fragment A in Scheme I is suspended in 4.0 equiv. of AgNO₃, then 7.0 equiv. of KCN, containing EtOH:THF:H₂0 (1:1:1), 0-25°C for 2 h to generate the C-methyl ester protected 15-methyl-LXA₄ analog that is concentrated and saponified in THF with LiOH (2 drops, 0.1 M) at 4°C 12-24 h to give the corresponding free acid.

### Synthesis of 16-dimethyl-LXA₄

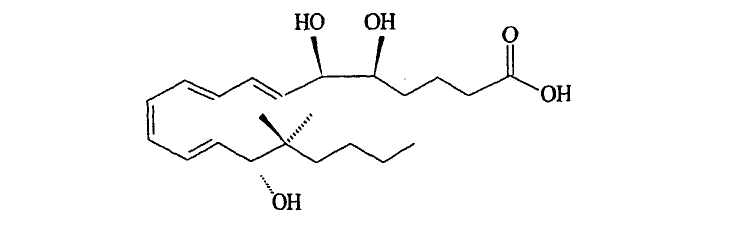

This compound is generated using the similar strategy by coupling d above with e vide supra, or f to generate the 15-phenyl-LXA₄ analog, or g to generate the 17-*m*-chlorophenoxy-LXA₄ analogs.

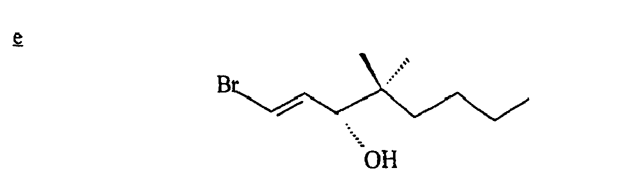

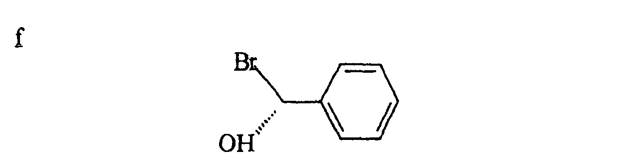

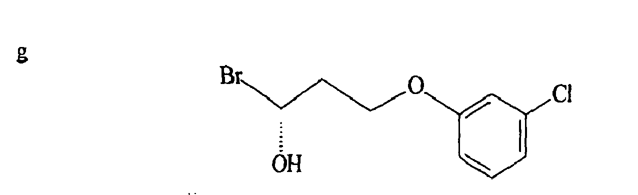

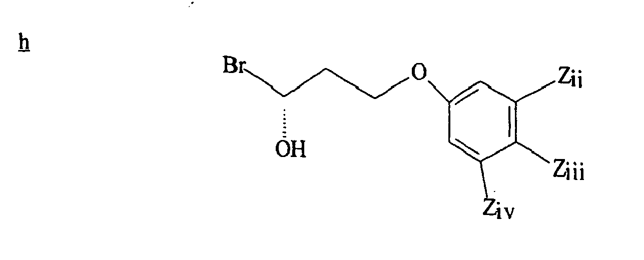

The appropriate C fragments in Scheme I (i.e. e, f, g, h, ) are each prepared as reviewed in Radüchel, B. and Vorbrüggen, H. (1985) Adv. Prostaglandin Thromboxane Leukotriene Res. 14:263 for the known corresponding prostaglandin analogues. In h, R=H; Cl, methoxy or halogen.

### Synthesis of 13,14-acetylenic-LXA₄ and halogen-containing analogs.

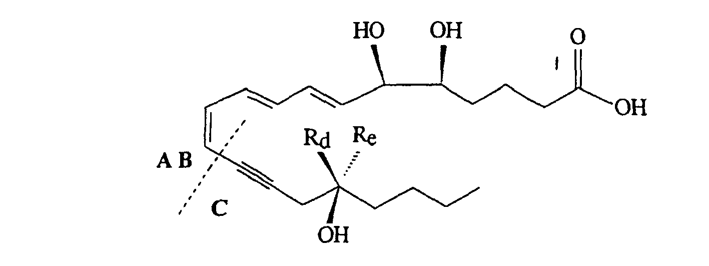

Using the A₂B₂ generated fragment from Scheme II, the corresponding C₂ fragments are prepared for coupling. Structures j and k are generated as in Nicolaou, K.C. et al. (1989) J. Org. Chem. 54:5527 and methylated as in Radüchel, B. and Vorbrüggen, H. (1985) Adv. Prostaglandin Thromboxane Leukotriene Res. 14:263 are coupled to 7 to yield these LX analogues. The materials may be subject to RP-HPLC for purification *vide supra*.

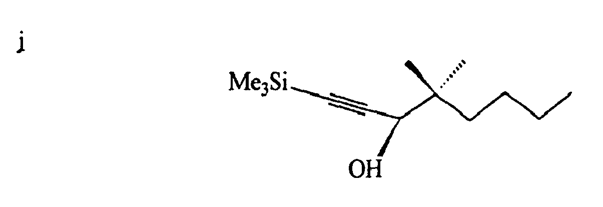

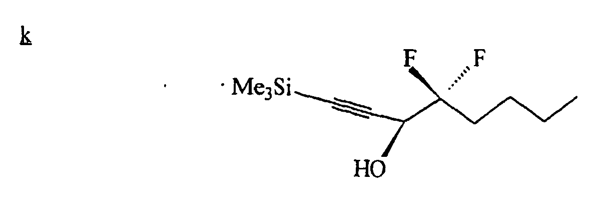

### Synthesis of 14,15-acetylenic-LXA₄ ,

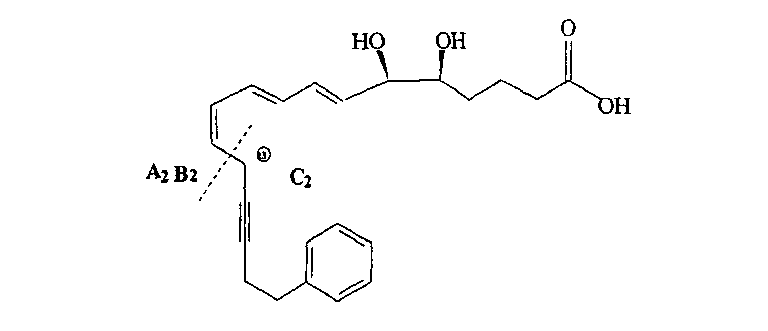

The designated combined A₂B₂ fragment can be prepared from couplings of fragments A₁ and B₁, illustrated in Route II to carry the structure of 7 or 4 vide supra for coupling to fragment C₂. The precursor for the C₂ fragment 1 can be prepared as in Radüchel, B. and Vorbrüggen, H. (1985) Adv. Prostaglandin Thromboxane Leukotriene Res. 14:263 for a prostaglandin analog.

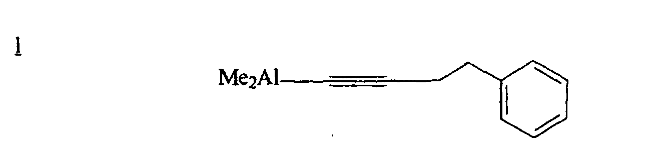

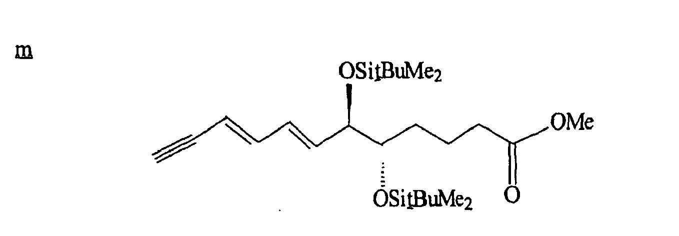

Precursor m as prepared previously (Nicolaou, K.C. (1989) J. Org. Chem. 54:5527) is added at 1.2 equiv. to 0.05 equiv. of Pd(PPh₃)₄, 0.16 equiv. of CuI, *n*-PrNH₂, in benzene with Me₂Al-carrying 1,2-3 h RT to yield n.

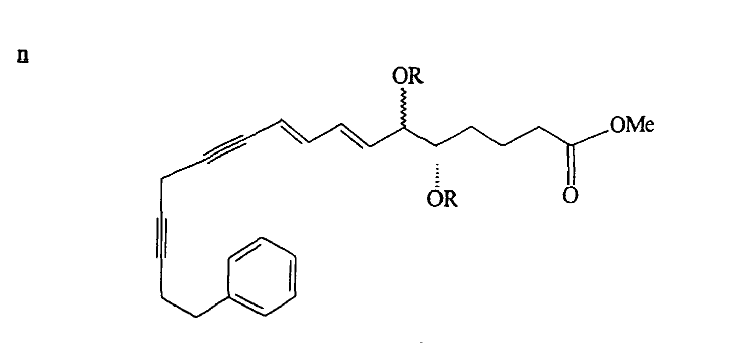

The alcohol protecting groups TBDMS=R are removed with 10 equiv. of HF-pyr, THF, 0-25°C (4 h) followed by exposure to 3.0 equivalents of Et₃N, MeOH, 25°C 15 min to open acid-induced δ-lactones that usually form between C-1-carboxy and C-5 alcohol in the lipoxins (Serhan, C.N. (1990) Meth. Enzymol.187:167 and Nicolaou. K.C. (1989) J. Org. Chem. 54:5527). After mild treatment with Lindlar cat. 5% by weight, the extracted material may be subjected to LiOH saponification in THF to generate the free acid of the target molecule that can be subject to further purification by RP-HPLC gradient mobile phase as in (Serhan, C.N. et al. (1990) Meth. Enzymol. 187:167).

### Synthesis of 15(±)methyl-cyclo-LXA₄

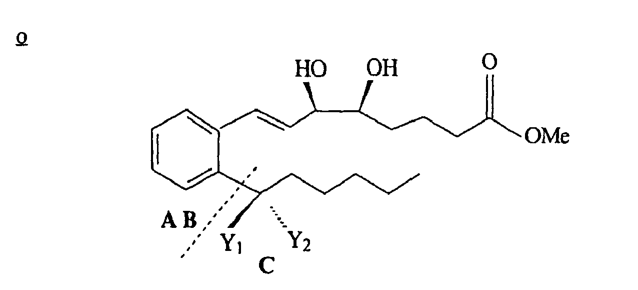

Compound o as the SiMe₃ derivative can be placed (∼ 1 gm) in a round bottom 100 ml flask under an atmosphere enriched with argon in degassed benzene (20 ml). To this add 3.0 equivalents of a vinyl bromide fragment *vide infra*. This coupling reaction is carried out in catalytic amounts of Pd (PPh₃)₄ and CuI and can be monitored by injected aliquots of this suspension into RP-HPLC monitored by UV abundance with a rapid scanning diode. The progression line course I-3 h at 23°C after which the material is extracted with ethyl acetate: H₂O 4:1 v/v) and concentrated by rotoevaporation. The methyl ester can be saponified in LiOH/THF to give quantitative yields of the free carboxylic acid. Other derivatives can be prepared as above using fragment A with different fragment B moieties that have been substituted to give for example a dimethyl or other derivative. This can be obtained by taking the readily available ketone p and treating it with CH₃MgBr (60°C) to generate q that can also be coupled to fragment A as above using conventional techniques such as Pd(O)-Cu(I) coupling. Increased chain length from C-15 can also be obtained.

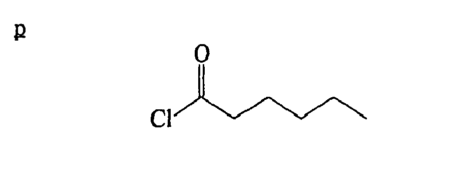

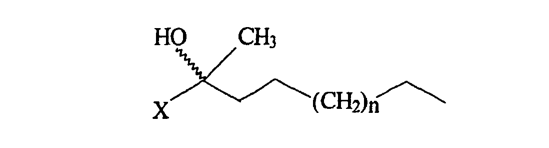

### Synthesis of 5-Methyl-LXB₄ and 4,4-Dimethyl-LXB₄.

The 5-methyl -LXB₄ hinders or retards 5-oxo-LYB₄ formation. Using the general scheme outlined above, the A fragment can be constructed to carry the 5-methyl in a vinyl bromide precursor that is coupled to a joined B + C fragment by Pd(0)-Cu(I) coupling.

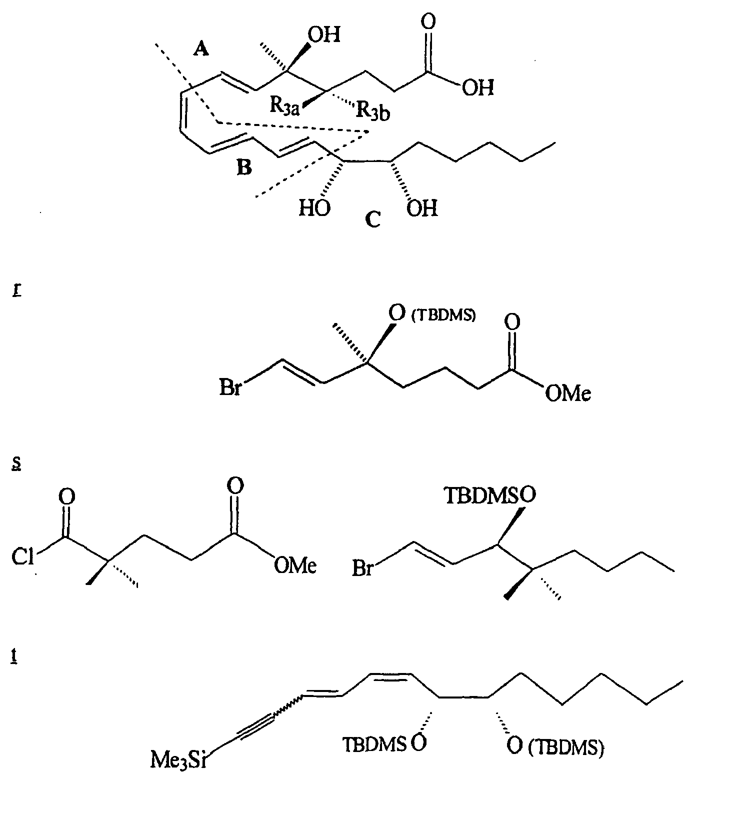

The vinyl bromide r can be obtained from the s that contains either dimethyl or hydrogen substituents at its C-4 position. The protected precursor t containing fragments B + C is generated as reported in reference (Nicolaou K.C. et al. (1991) Angew. Chem. Int. Ed. Engl. 30: 1100-16.). Compound t is converted to s or 28 by coupling with the indicated vinyl bromide. Thus the target molecule can be generated by adding r at 1.0 equv. (≈ 1 gm) to a round bottom flask degassed containing Et₂NH as solvent with 1 injected in Et₂NH at 1.2 equiv. Pd(Ph₃P)₄ is added at 0.02 equiv. to give the 8(9)-containing acetylenic precursor methyl ester of s.

The material is extracted and subject to rotoevaporation suspended in quinoline (0.5 eq) in CH₂Cl₂ and subject to hydrogenation using (10%; 25°C) Lindlar catalyst and a stream of H₂ gas to selectively reduce the acetylenic double bond at position 8. The formation of the tetraene component of the methylester of 5-methyl-LXB₄ or 4-dimethyl-LXB₄ methyl ester can be monitored by RP-HPLC to assess completion of the reduction (i.e., I-3h). The methyl#esters are next saponified to their corresponding free acids by treating the products with LiOH in THF 25µl H₂O added at 0⇒24°, 8 - 24h.

### Example 2 Lipoxin A₄ Metabolism by Human Promyelocytic Leukemia Cells and Monocytes : Half-life Assay

HL-60 cells were purchased from American Type Culture Collection (Rockville, MD), and other cell culture reagents were from GIBCO (Grand Island, NY). Versene (EDTA) was from Whittaker Bioproducts (Walkersville, MD). Synthetic 11,12-acetylenic LXA₄ methyl ester and lipoxins were from Cascade Biochemical (Reading, U.K.). 15(S)-15-*m*-PGE₁, PGE₁ and 5-HETE were from Cayman Chemical Co. (Ann Arbor, MI). [11,12-³H]LXA₄ was prepared from 11,12-acetylenic LXA₄ using Lindlar catalyst as a custom tritiation (NET-259, lot 0 2793-275, New England Nuclear, Boston, MA). Tritiated products were isolated using RP-HPLC (Fiore et al. (1992) J. Biol. Chem. 267:16168; Serhan, C.N. (1990) Meth. Enzymol. 187:167). Methoxyamine and NAD were from Sigma Chemical Company (St. Louis, MO). Manganese dioxide and Adams reagent were from Aldrich Chemical Co. (Milwaukee, WI).

Human poymorphonuclear cells (PMN) were obtained from healthy volunteers by gradient centrifugation of heparinized fresh venous blood (Böyum, A. (1986) Scand. J. Clin. Lab. Invest. 21:77). HL-60 cells were seeded in RPMI supplemented with penicillin (100 U/ml), streptomycin (100 µ/ml), fetal bovine serum (10%) (Hyclone, Logan, UT) and incubated (37°C with 5% CO₂ atmosphere) in plastic 250 ml flasks. Individual flasks containing 5 x 10⁻⁷ HL-60 cells/ml were incubated in the presence or absence of phorbol 12-myristate 13-acetate (PMA) (10 or 16 nM, 24-27 h) and adherence was monitored for induction of macrophage-like phenotype as in Collins, S.J. (1987) Blood 70:1233. Peripheral blood monocytes were obtained (Goldyne, M.E. et al. (1984) J. Biol. Chem. 259:8815 after plating fresh mononuclear cells onto plastic petri dishes containing PBS with glucose (1 mg/ml) for 1 h at 37°C. Non-adherent cells were removed and adherent mononuclear cells; were gently resuspended using Versene (7 ml/plate) and washed in PBS. PMN (>98%), adherent monocytes (>95%) and HL-60 cells were enumerated by light microscopy, suspending in PBS for incubations, and <2-3% in each case were permeable to trypan blue. For some experiments, cell-free supernatants were prepared from HL-60 cells treated with PMA (16 nM) for 24 - 72 h. After harvesting, the differentiated cells were washed, then subject to freeze-thaw lysis (repeated 3 times) and ultracentrifugation (100,000 g, 1 h).

Incubations with eicosanoids were stopped with cold methanol containing either PGB₂ or 5-HETE as internal standards (5-HETE was used when 15-oxo-ETE was quantified). Products were extracted using Sep-pak C18 and routinely chromatographed as in Serhan , C.N. (1990) Meth. Enzymol. 187:167. RP HPLC system consisted of an LKB gradient dual pump equipped with an Altex Ultrasphere-ODS (4.6 mm x 25 cm) column, flow rate 1 ml/min eluted (0-20 min) with methanol/H₂O/acetic acid (65:35:0.01) and methanol/acetic acid (99.99/0.1) in a linear gradient (20-45 min) that was used to quantitate the ω-metabolites of LTB₄ (i.e. 20-COOH and 20-OH-LTB₄) as well as LXA₄. Recovery of internal standards was 82.2 7.9, mean S.D. (n=13). Compounds I-IV were separated using an Altex Ultrasphere-ODS column (10 mm x 25 cm) eluted at a flow rate of 3.0 ml/min with methanol/H₂O/acetic acid (60:40:0.01, v/v/v). Formation of 15-oxo-ETE by 100,000 g supernatants (cf. Agins, A.P. et al. (1987) Agents Actions 21:397; Xun, C-Q. et al. (1991) Biochem. J. 279:553; and Sok, D-E. et al. (1988) Biochem. Biophys. Res. Commun. 156:524) was quantified after RP-HPLC using an ODS column (4.6 mm x 25 cm) eluted with methanol/H₂O/acetic acid (70:30:0.01, v/v/v) monitored at 280 nm with a flow rate of 1 ml/min. Monocyte-derived products were also chromatographed using a Hypersil column (5 IL, 4 mm x 300 mm) eluted with methanol/H₂0/acetic acid (60.40:0.01, v/v/v) and a flow rate of 1 ml/min. On-line spectra were recorded using a diode array detector (Hewlett-Packard 1040M series II) equipped with HPLC^{3D} ChemStation software (DOS series). Spectra were acquired using step 4 nm, B_{w} = 10 nm, range = 235-360 nm with a sampling interval of 1.28 sec.

GC/MS was performed with a Hewlett-Packard 5971 A mass selective detector quadrupole equipped with a HPG1030A workstation and GC 5890. The column was a HPUltra 2 (cross-linked 5% phenyl methyl silicone gum phase; 25 m x 0.2 mm x 0.33 µm) and injections were made in the splitless mode in bis(TMS)trifluoroacetamide (BSTFA). The temperature program was initiated at 150 °C and reached 250°C at 10 min and 325° at 20 min. Standard saturated fatty acid methyl esters C₁₆-C₂₆ gave the following retention times (min:sec; mean of n=6). C₁₆, 8.03; C₁₈, 9.77; C₂₀, 12.22; C₂₂, 16.11; C₂₄, 20.72; C₂₆, 23.62 that were used to calculate respective C values of LX-derived metabolites as in Serhan, C.N. (1990) Meth. Enzymol. 187:167. Diazomethane was prepared and the methyl ester products were treated with BSTFA (Pierce Chemical Co., Rockford, IL) to obtain Me₃Si derivatives. Methyl ester *O*-methoxime derivatives were prepared as in Kelly, R.W. and Abel, M.H. (1983) Biomed. Mass Spectrom. 10:276. Catalytic hydrogenations were performed in methanol (1 ml) with Adams reagent (Aldrich, Milwaukee, WI) by saturating the platinum IV oxide (I-2 mg) with a stream of bubbling hydrogen (20 min, RT). After extraction, materials were treated with diazomethane followed by BSTFA (overnight; RT).

### RESULTS

Metabolism of LXA₄: Intact neutrophils from peripheral blood of healthy donors did not significantly metabolize exogenous LXA₄ while cells from the same donors rapidly transformed LXA₄ via ω-oxidation. In contrast, PMA-treated HL-60 cells that displayed monocyte/macrophage-like characteristics rapidly transformed LXA₄. Within the first 60 s of exposure, > 70% of LXA₄ was metabolized. In the absence of PMA treatment, neither intact HL-60 cells (undifferentiated) nor their cell-free supernatants (100,000 x g) ft=orm LXA₄ (n=3).

Differentiated HL-60 cells incubated with LXA₄ converted this eicosanoid to several products. Labeled LXA₄ was transformed to four main products that carried tritium (denoted compounds I-IV), which were collected for further analysis.

Structures of compounds I-IV To obtain quantities of these compounds enabling structural studies, their retention times in RP-HPLC were established using the ³H-label elution profile to mark boundaries, and unlabeled samples pooled from several incubations were chromatographed and individually collected from within these regions for GC/MS analysis. Selected ion monitoring of the products obtained after treatment with diazomethane and BSTFA revealed that compounds I-IV each displayed prominent ions at m/z 203 [-CH(OSiMe₃)-(CH₂)₃-COOCH₃] indicating that carbons 1 through 5 of LXA₄ (carboxylic carbon is number 1) were not modified, although each product gave a different retention time than LXA₄. The methyl ester, trimethylsilyl derivative of LXA₄ displayed prominent ions in its electron impact spectrum at m/z 203 (base peak) and 173, with its molecular ion at 582 (M⁺4). Other ions of diagnostic value in this derivative of LXA₄ are observed at m/z 171 (203-32), 409 (M-173), 379 (M-203), 482 (M-100) and 492 (M-90) (Serhan, C.N. et al. (1984) Proc. Natl. Acad. Sci. USA 81:5335; and Serhan, C.N. (1990) Meth. Enzymol 187:167. It is noteworthy that the lipoxins in general are known to give extremely weak molecular ion peaks (Serhan, C.N. et al. (1984) Proc. Natl. Acad. Sci. USA 81:5335). Nevertheless, compounds labeled I & II also possess prominent ions at m/z 173 (Me₃SiO⁺=CH-(CH₂)₄-CH₃) indicating that the carbon 15-20 fragment of these LXA₄-derived products was intact, while the ion at m/z 173 was not evident in compounds III and IV. Thus, the conclusion that compounds I-IV are metabolites of LXA₄ was based upon: their physical properties (HPLC and GC/MS), the finding that they carry tritium label, as well as the absence of these products in incubations with HL-60 cells not treated with PMA.

Next, compounds III and IV were focused on since it appeared that they represent metabolites with structural modifications in the carbon 15 through 20 fragment of LXA₄. Since ω-oxidation (hydroxylation at carbon 20) was a possibility, ions that could result from the respective 20-OH and 20-COOH forms of LXA₄ after derivatization, namely m/z 261 and 217 (Me₃SiO⁺=CH-(CH₂)₄-CH₂OSiMe₃ and Me₃SiO⁺=CH-(CH₂)₄-CO₂Me), were scanned in the acquired GC-MS data profiles. Neither III nor IV displayed prominent ions at either m/z 261 or 217 indicating that these products were not likely the result of ω-oxidation.]

The mass spectrum (C value 24.3) of the Me₃Si derivative, methyl ester of compound III was obtained. Prominent ions in its spectrum were observed at m/z 203 (base peak, CH(OSiMe₃)-(CH₂)₃-COOCH₃), 171 (203-32; elimination of CH₃0H), 215 [(M-203)-90, elimination of trimethylsilanol (Me₃SiOH)] and 99 (O=C-(CH₂)₄-CH₃). Ions of lower intensity were a m/z 508 (M⁺) and 418 (M-90; loss of Me₃SiOH). The presence of these ions suggested that the material that coeluted with ³H-labeled compound III was the 15-oxo-derivative of LXA₄. This is supported by several lines of evidence, namely the virtual loss of the prominent ion at m/z 173 (Me₃SiO⁺=CH-(CH₂)₄-CH₃), presence of m/z 99 (O=C₄CH₂)₄-CH₃), the absence of a tetraene chromophore and appearance of a new chromophore at UV λₘₐₓ at 335-340 nm. The tetraenone chromophore was confirmed by treating LXA₄ with MnO₂ in chloroform as used for prostaglandin conversion (Änggard, E. and Samuelsson, B. (1964) J. Biol. Chem. 239:4097). Also, the mass spectrum of the catalytic hydrogenation product gave a C value of 25.1 with prominent ions at m/z 203 (base peak), m/z 99 (66%), m/z 313 (M-203 or M-CH(OSiMe₃)-(CH₂)₃-COOCH₃; 35%) and m/z 171 (36%) with no prominent ion at m/z 173. Less intense ions were at m/z 516 (M⁺) and m/z 426 (M-90). Thus, the upward shift of 8 amu and framentation of this saturated derivative were consistent with the generation of the corresponding 15-oxo-derivative.

To examine this LXA₄-derived product further, an aliquot of the material eluting beneath the peak labeled III was treated with diazomethane followed by methoximation (as in Bergholte, J.M. et al. (1987) Arch. Biochem. Biophys. 257:444) and treatment with BSTFA. Its spectrum, C value 25.4, showed prominent ions at m/z 203 (base peak), 171 (203-32; loss of CH₃OH) and 229 [M-128 or CH₃O-N=C-(CH₂)₄CH₃-(2x90)]. Ions of lower intensity were at m/z 537 (M⁺), 466 (M-71, the αt-cleavage ion M-CH₂(CH₂)₃CH₃), 481 (M-56 or M-CH₂=CH-CH₂-CH₃, a McLafferty rearrangement ion), 431 [M-106 (possibly loss of C₇H₅N⁺)]. 401 [M-136 (elimination of Me₃SiOH + CH₃ +. OCH₃) and 460 (M-77, loss of NOCH₃ plus MeOH). Again, an ion at m/z 173 that would have originated from an alcohol-containing C-15 fragment (Me₃SiO⁺-CH-(CH₂)₄-CH₃) was virtually absent in its spectrum. Thus, the ions present are consistent with the methyl ester, *O*-methoxime derivative generated from the 15-oxo-containing derivative of LXA₄. Together the prominent ions observed with these different derivatives suggest that material eluting beneath the peak labeled III was the 15-oxo- product of LXA₄ (i.e. 15-oxo-LXA₄).

The mass spectrum of the methyl ester Me₃Si derivative (C value 26.0) of compound IV showed. prominent ions at m/z 203 (base peak, CH(OSiMe₃)-(CH₂)₃COOCH₃), 171 (203-32; loss of CH₃OH), 99 (O=C-(CH₂)₄-CH₃) and 307 (M-203 or MCH(OSiMe₃)-(CH₂)₃-COOCH₃). Ions of lower intensity were at m/z 510 (M⁺), 420 (M-90, loss of trimethylsilanol) and 208 (M-(99+203)). Its UV spectrum showed a triplet of absorbance with maxima at 259, 269 and 280 nm, consistent for a conjugated triene chromophore. The presence of these ions and UV spectrum suggest that IV was a dihydro-15-oxo-metabolite of LXA₄. This basic structure was supported by the presence of the ion at m/z 99 that is consistent with a keto group at position carbon 15, and the presence of m/z 203 as base peak revealed that the alcohol groups at carbons 5 and 6 remain intact. In addition, the absence of a trienone chromophore (λ cal = 310 nm) indicates that loss of a double bond was at Δ13-14 position to give the observed triene chromophore. Together these results indicate that compound IV was 13,14-dihydro-15-oxo-LXA₄.

The methyl ester, Me₃SiO derivative of compound II (C value - 25.4) gave ions at m/z 203 (base peak; CH(OSiMe₃)-(CH₂)₃COOCH₃), 173 (Me₃SiO+=CH-(CH₂)₄-CH₃), 171 (203-32) and 584 (M⁺). Its molecular ion was two mass units higher than the LXA₄ derivative. These ions and a triplet band of absorbance λₘₐₓMeOH 259 nm, 269 and 282 nm suggest that compound II was a dihydro-derivative of LXA₄. The methyl ester, Me₃SiO derivative of compound I from HL-60 cells gave two products in GC. The major one (C value = 25.0) gave similar ions in its mass spectrum as LXA₄, but instead its molecular ion was at m/z 586 with ions also present at m/z 555 (M-31) and 496 (M-90), indicating that two of the four double bonds were reduced (not shown). However, identical products were not observed with peripheral blood monocytes (*vide infra*), and thus the HL-60 cell-derived materials from peak I were not further characterized in the present experiments. The structures of I-IV and results from Fig. I indicate that LXA₄ is not metabolized by ω-oxidation by intact leukocytes but instead is both dehydrogenated at carbon 15 alcohol and also transformed from a conjugated tetraene to triene structures. Taken together these observations suggested that LXA₄ may be attacked by NAD-dependent 15-prostaglandin dehydrogenase (5-PGDH), an enzyme known to carry out similar reactions with prostanoids as substrate (Änggard, E. and Samuelsson, B. (1964) J. Biol. Chem. 239:4097, and reviewed in Hansen, H.S. (1976) Prostaglandins 12:647).

15-PGDH activity was recently shown to be induced in HL-60 cells (Xun, C-Q. et al. (1991) Biochem. J. 279:553), and it apparently utilizes 15-HETE as substrate with 92% efficiency compared to PGE₂ (Agins, A.P. et al. (1987) Agents Actions 21:397). Indeed, 100,000 g supernatants prepared from PMA-treated HL-60 cells converted 15-HETE to 15-oxo-ETE indicating the presence of a dehydrogenase activity after differentiation. LXA₄ competed for catalysis of 15-HETE giving a Kᵢ = 8.2 ± 2.6 µM (S.E.M., n=6) calculated from Lineweaver-Burke plots. At equimolar concentrations of LXA₄ and 15-HETE, LXA₄ blocked 15-oxo-ETE formation by ≈50%. The relative conversion for LX compared to PGE₁ by 100,000 g supernatants (Fig. 5) indicated that LXA₄, 11*-trans*-LXA₄ as well as LXB₄ but not 15-methyl-PGE₁ were converted. Together, these results suggest that LXA₄ > 11-*trans*-LXA₄ > LXB₄ are substrates for 15- PGDH or an equivalent enzyme system.

Since PMA induces differentiation to monocyte-macrophage-like lineage of HL-60 cells (Collins, S.J. (1987) Blood 70:1233), peripheral blood monocytes were incubated to determine if they metabolize lipoxin. Lipoxins display potent actions with monocytes (Stenke, L. et al. (1991b) Biochem. Biophys. Res. Commun. 180:255) and these cells do not ω-oxidize eicosanoids (Goldyne, M.E. et al. (1984) J. Biol. Chem. 259:8815). When suspensions of both intact monocytes (n=5) and permeabilized cells (freeze-thaw or saponin-treated, n=5) were exposed to LXA₄, it was converted to 15-oxo-LXA₄ and conjugated triene-containing products, 13,14-dihydro-LXA₄ and 13,14-dihydro-15-oxo-LXA₄ (see Table 1). As with differentiated HL-60 cells, monocytes rapidly converted LXA₄ (> 60%) within 30 s (Fig. 6). The temporal relationships for formation of these metabolites in both intact and permeabilized monocytes were similar and suggest that 15-oxo-LXA₄ metabolite is a transient intermediate. Also, in each monocyte suspension incubated with ³H-LXA₄ (d=33), 13,14-dihydro-15-oxo-LXA₄ and 13,14-dihydro-LXA₄ were major products carrying radiolabel. It is noteworthy that a product eluting before 13,14-dihydro-LXA₄ at 15.5-17 min was observed that also displayed a triene chromophore and was likely the 11-*trans* isomer of 13,14-dihydro-LXA₄ that results from *cis-trans* isomerization encountered during work-up, The 11-cis double bond of native LXA₄ is labile and readily isomerizes to all*-trans* during extraction and isolation (Romano, M. and Serhan, C.N. (1992) Biochemistry 31:8269).

### Example 3 Binding Affinity to Lipoxin Receptors Analogs

Human promyelocytic leukemia cells (HL-60) were purchased from the American Type Culture Collection (Rockville, MD). RPMI medium and cell culture reagents were from GIBCO (Grand Island, NY). Synthetic LXA₄, trihydroxyheptanoic acid (methyl ester), LXB₄, LTD₄, LTC₄ and LTB₄ were from Biomol (Plymouth Meeting, PA), and SKF 104353 was from Smith Kline and French Laboratories. ONO 4057 was from ONO Pharmaceutical Co., Ltd. (Osaka, Japan). [14,15-³H]LTB₄ (32.8 mCi/mmole), [1-¹⁴C]arachidonic acid (50.2 mCi/mmole), ³²PγATP (3,000 Ci/mmole), [9,10⁻³H(N)]palmitic acid (30.0 mCi/mmole), and [9,10- ³H(N)]myristic acid (30.7 Ci/mmole) were purchased from New England Nuclear (DuPont Co., Boston, MA). 11,12-acetylenic LXA₄ was from Cascade Biochemicals (Oxford, UK). Microcentrifuge tube filters (0.45 µm cellulose acetate) were purchased from PGC Scientific (Gaithersburg, MD) and silicon oils were from Harwick Chemical Corp. (Akron, OH) (d=1.05) and Thomas Scientific (Swedesboro, NJ) (d=0.963), respectively. Nitroblue tetrazolium, PMA, DMSO, proteases, retinoic acid and Actinomycin D were purchased from Sigma (St. Louis, MO). Islet activating protein (IAP) was from LIST Biological Lab., Inc. (Campbell, CA). Plasticware, Whatman LK6D TLC plates and solvent (HPLC grade) were from Fisher (Springfield, NJ).

Preparation of [11,12-³H]LXA₄. Tritiation of 11,12-acetylenic LXA₄ methyl ester was carried out under a custom tritiation service (NET-259:92-2326) by New England Nuclear (Boston, MA). Briefly, 11,12- acetylene methyl ester was characterized by UV absorbance and reversephase HPLC as in (Nicolaou, K.C. et al. (1985) J. Am. Chem. Soc. 107:7515) and exposed to tritium atmosphere in methylene chloride at room temperature. This incubation was stirred in the presence of Lindlar catalyst (1.0 mg from Fluka Chemicals) for ∼ 1 h. The resulting mixture was stored in methanol and isolated using RP-HPLC. Tritiated products were chromatographed as methyl esters utilizing a gradient HPLC system equipped with a photodiode array rapid spectral detector (Serhan, C.N., Methods in Enzymology: Arachidonate Related Lipid Mediators, in Murphy R.C., Fitzpatrick, F. (eds.), vol. 187. Orlando, FL, Academic, (1990), p. 167). This mixture contained both [11,12-³H]LXA₄ and [11,12-³H]-11-*trans*-LXA₄ methyl esters (∼ 1:3 ratio) as determined by coelution with synthetic standards. After RP-HPLC, fractions containing [11,12-³H]LXA₄ were collected, and extracted into ethyl acetate. The free acid was prepared by LiOH saponification (Fiore, S. et al. (1992) J. Biol. Chem. 267:16168). Material from these fractions, when injected into UV-electrochemical detection HPLC, gave greater than 90% of radioactivity associated with a tetraene-containing product that coeluted with synthetic LXA₄. Materials that eluted with the retention time of authentic LXA₄ in two HPLC systems were taken for binding experiments. The specific activity calculated for [11,12- ³H]LXA₄ was 40.5 Ci/mmole.

Cell cultures and differentiation. HL-60 cells were seeded in RPMI medium supplemented with 100 U/ml penicillin, 100 µg/ml streptomycin, and 10% fetal calf serum (Hyclone, Logan, UT), incubated at 37°C with 5% CO₂ atmosphere in 250 ml flasks. Individual flasks containing ∼ 50 x 106 cells/ml next received dimethyl sulfoxide (DMSO) (1.12% v/v. 120 h) or retinoic acid (RA) (1 µM, 120 h) or phorbol myristate acetate (PMA) (20 nM, 48 h). Before performing binding assays, cells were washed twice in phosphate buffered saline (PBS), Ca²⁺- and Mg²⁺- free, enumerated and suspended at 20 x 10⁶ cells/ml in Tris buffer (10 mM), pH 7.4 (Fiore, S. et al. (1992) J. Biol. Chem. 267:16168). Nitro blue tetrazolium reduction was performed to monitor induction of polymorphonuclear phenotype as in (Imaizumi, M. and Breitman, T.R. (1986) Blood 67:1273), and cell adherence was determined for induction of macrophage-like phenotype (Collins, S.J. (1987) Blood 70:1273). Human umbilical vein endothelial cells (HUVEC) maintained in culture third passage were obtained from Dr. M. Gimbrone (Brigham and Women's Hospital Department of Pathology).

### PMN, platelet and RBC isolation from peripheral blood.

Human PMN were obtained by the modified Böyum method (Böyum, A. (1968) Scan. J. Clin. Lab. Invest. 21:77) from fresh heparinized blood after venipuncture of healthy normal volunteers. Suspensions in PBS were monitored for cell number and viability by their ability to exclude trypan blue both exceeding 98%. Red blood cells were obtained from 10 ml of heparinized blood after three centrifugations in PBS (2,500 rpm, 10 min at 21 °C). Blood drawn in acidic citrate dextrose (9:1, v/v) was used to isolate platelets as previously described (Serhan, C.N. and Sheppard. K.-A. (1990) J. Clin. Invest. 85:772).

Ligand binding. ³H-LXA₄ and ³H-LTB₄ binding was performed essentially as in Fiore, S. et al. (1992) J. Biol. Chem. 267:16168. After suspending cells in Tris buffer 10 mM (pH 7.4, Ca²⁺ 2.5 mM, Mg²⁺ 1.2 mM), aliquots (0.5 ml) were incubated (20 min at 4°C) with ³H-ligands alone (0.3 nM) or in the presence of increasing concentrations of homoligands or other compounds (3-300 nM). The incubations were rapidly centrifuged (60 sec, 12,000 g) on silicon oil (d=1.028), and cell-associated radioactivity was determined by liquid scintillation counting (Wallac 1409, Pharmacia, Piscataway, NJ). Binding experiments with HUVEC cells were performed in 12 well plates with 3.5 x 10⁵ cells/well. After 10 min, wells were washed twice with PBS and cell-associated label was recovered by adding glacial acetic acid (0.5 ml). Results obtained from these assays were submitted for further analysis using the Ligand program (Elsevier-Biosoft, Cambridge, UK).

PLD activity. Human PMN and HL-60 cells (50 x 10⁶ cells/ml) prepared as above were incubated with ³H-myristic acid or ³H-palmitic acid (8 µCi/50 x 106 cells) for 40-60 min at 37°C in PBS. Cell uptake ranged between 60-80% of added label, 7.1 ± 4.2% (n=10; mean ± S.D.) and 32.6 ± 10.3% (n=6; mean ± S.D.) into total phospholipid classes of PMN and HL-60 cells, respectively. Incubations were carried out at 37°C (10 x 10⁶ cells/ ml PBS). Agonists were added in 50 µL PBS or with 1:10 (v/v) EtOH:PBS for phosphatidylethanol (PEt) formation as in Billah, M.M. et al. (1989) J. Biol. Chem. 264:17069. At indicated times, incubations were stopped by adding 3.5 ml of ice cold CHCl₃/MeOH (2/5, v/v) containing 1- ¹⁴C-arachidonic acid (5,000 cpm) used here as internal standard to quantitate extraction recoveries. Samples were extracted using a modified Bligh and Dyer extraction as in (Serhan, C.N. and Sheppard, K.-A. (1990) J. Clin. Invest. 85:772). Organic phases, concentrated in 50 µL of CHCl₃/MeOH (8/2, v/v), were spotted onto linear K6D TLC plates developed with the organic phase of ethyl acetate/isooctane/acetic acid/water (110/50/20/100, v/v/v/v) for 50 min (Billah, M.M. et al. (1989) J. Biol. Chem. 264:17069), In this system, phosphatidic acid (PA) gave an R_{f} = 0.1 ± 0.04 and PEt gave an R_{f} = 0.56 ± 0.04; n = 38 ± S.D. that were clearly separated from other phospholipids (that remained at the origin) or neutral lipids (R_{f}= 0.75-0.90). Lipids were visualized with iodine vapor and identified by co-elution with authentic standards that were also spotted and chromatographed in each TLC plate. Regions corresponding to PA, PEt, and internal standards were scraped and quantified by liquid scintillation counting. In addition to ³H-myristate or ³H-palmitate labeling, both [1-¹⁴C]arachidonate (0.25 µCi/30 x 10⁶ PMN) and ³²PγATP (20 µCi/50 x 10⁶ PMN) labeled cells were used to monitor LXA₄₋stimulated formation of PA and PEt generation. In these experiments, PA was resolved using ethyl acetate/isooctane/acetic acid (45/15/10, v/v/v) as solvent system (Bocckino, S.B. et al. (1989) Anal. Biochem. 180:24) and gave an R_{f} = 0.46 ± 0.03 (n = 15). All values reported for PEt formation in Tables 4 and 5 were calculated by subtracting the dpm obtained in the presence of agonist(s) alone minus those measured in the presence of agonist(s) and 0.5% EtOH.

Impact of IAP and staurosporine on LXA₄-induced PLD activity. PLD activity assays, performed as described (*vide supra*), were preceded by cell exposure to either IAP or staurosporine. IAP treatment of PMN was performed as previously described (Nigam, S. et al. (1990) J. Cell Physiol. 143:512), and HL-60 cells were incubated in the presence or absence of IAP (300 ng/ml) for 2 h at 37°C (Kanaho, Y. et al. (1992) J. Biol. Chem. 267:23554). Aliquots (10⁷ cells/0.5 ml) were added to 0.4 ml buffer. Next, incubated cells were exposed to either 100 µl of vehicle (PBS, EtOH 0.04%) or LXA₄ (10⁻⁷ M and 10⁻⁹ M), in the presence or absence of 0.5% EtOH. Staurosporine (100 nM) was added to cell suspensions for 5 min at 37°C before addition of LXA₄.

### RESULTS

After preparation of synthetic [11,12-³H]LXA₄, its specific binding to promyelocytic cells (HL-60) was characterized and direct comparisons with specific binding of [14,15-³H]LTB₄ were performed. When routine phenotypic markers were monitored, untreated HL-60 cells displayed a low level of specific binding for both ³H-LXA₄ and ³H-LTB₄ ligands. Differentiation induced by exposure to either DMSO (1.12%) or retinoic acid (1 µM) for 5 days was accompanied with a three to fivefold increase in specific binding for both radioligands. PMA-treated cells (20 nM, 48 h) displaying characteristics of macrophagic-like phenotype, i.e. NBT negative cells that adhere to plastic (see Imaizumi, M. and Breitman, T.R. (1986) Blood 67:1273; Collins, S.J. (1987) Blood 70:1233), also led to the appearance of specific binding with both ³H-LXA₄ and ³H-LTB₄. Equilibrium binding with ³H-LXA₄ at 4°C was reached at 10 min that remained virtually unchanged for the next 20 min.

To assess whether induction of specific binding for both ³H-ligands required *de novo* synthesis, Actinomycin D (2 µg/ml) was added with PMA incubations. Actinomycin D blocked the PMA-induced increment in specific binding for both labeled eicosanoids, suggesting inhibition of *de novo* protein biosynthesis also blocked appearance of specific binding sites. The impact of protease and glycosidase treatments was assessed with both differentiated HL-60 cells and human PMN for ³H-LXA₄ specific binding. Protease treatment reduced specific binding and provided additional evidence in support of a protein component of LXA₄ specific binding sites.

Results from isotherm binding assays (4°C, 10 min) with differentiated HL-60 cells and [11,12-³H]LXA₄ (0.1-30 nM) showed that [11,12-³H]LXA₄ specific binding sites gave a K_{d} = 0.6 ± 0.3 nM. A nonlinear portion of the Scatchard plot was observed for concentrations observed with LXA₄ specific binding with human PMN (Fiore, S. et al. (1992) J. Biol. Chem. 267:16168). Results obtained here with LTB₄ specific binding with HL-60 cells, K_{d} = 0.12 nM, are essentially in agreement with values recently reported by Harada (Xie, M. et al. (1991) J. Clin. Invest. 88:45), namely K_{d} = 0.23 nM.

To further characterize the interactions of ³H-LXA₄ with its specific binding sites, competition binding experiments were performed with differentiated HL-60 cells. LXA₄, LXB₄. LTB₄, LTC₄ and the leukotriene receptor antagonists SKF 104353 (LTD₄ antagonist; Harada, Y. (1990) Hiroshima J. Med. Sci. 39:89) and ONO-4057 (LTB₄ antagonist; Gleason. J.G. et al. (1987) J. Med. Chem. 30:959) were assessed as potential competing ligands. Neither LXB₄, LTB₄, nor trihydroxyheptanoic acid (methyl ester) (300 nM) were able to displace ³H-LXA₄ specific binding with differentiated HL-60 cells while LTC₄ caused ∼30% decrease of specific binding when added in 3 log molar excess. The finding that LXA₄ (300 nM) was unable to compete with ³H-LTB₄ (0.3 nM) binding to differentiated HL-60 cells suggests that LXA₄ and LTB₄ interact with separate classes of specific binding sites. Leukotriene receptor antagonists SKF 104353 and ONO-4057 did not displace ³H-LXA₄ binding with differentiated HL-60 cells, but SKF 104353 and LTD₄ were effective in competing the specific ³H-LXA₄ binding with HUVEC. HUVEC displayed a K_{d} of 11.0 ± 2.6 nM and a Bₘₐₓ of 2.5 x 10-¹⁰ M for ³H-LXA₄, and virtually identical values were calculated for LTD₄ competition. In the case of ³H-LTB₄, HUVEC did not specifically bind LTB₄, but non-specific cell association was evident with this ³H-ligand (n=3; not shown). Specific association of ³H-LXA₄ was not evident among several other cell types surveyed. Here, neither washed platelets, RBCs, the β-cell (Raji), nor T-cell (Jurkat) cultured cell lines displayed specific binding for ³H-LXA₄. Taken together, these results indicate that LXA₄ interacts with unique binding sites in differentiated HL-60 cells that is not sensitive to either leukotriene receptor antagonist (SKF 104353 or ONO-4057). In HUVEC, ³H-LXA₄ specific binding was sensitive to both LTD₄ and SKF 104353 but not ONO-4057, suggesting that ³H-LXA₄ specific binding in this cell type may reflect its interaction with putative LTD₄ receptors.

LXA₄ rapidly stimulates phosphatidic acid formation in human neutrophils (Nigam, S. et al. (1990) J. Cell Physiol. 143:512). To determine if ³H-LXA₄ binding confers PLD activation, PEt and PA were monitored in both PMN and HL-60 cells. Results indicated that LXA₄ stimulates PLD activity in these cell types with similar temporal responses. PMN exposed to LXA₄ (10⁻¹⁰ M) rapidly generated the ethanol trapping product PEt within 60 s that declined to baseline levels by 5 min. In the absence of added ETOH, PEt was not formed at statistically significant levels. A biphasic concentration dependence was obtained for PEt formation in both PMN and differentiated HL-60 cells. An apparent maximal response was noted with the concentration range of ∼10⁻⁹-10⁻¹⁰ M LXA₄, and a second peak of activity was observed at 10⁻⁷ M LXA₄. Below 10⁻⁸ M, both the chemotactic peptide FMLP and LXA₄ gave results of similar magnitude with FMLP appearing to be slightly more potent with PMN from some donors. To evaluate the potential contribution of other biosynthetic pathways, LXA₄-induced PA formation was also examined in both ³²PγATP and [1-¹⁴C]-arachidonate-labeled PMN. ³²P-labeled PA was evident in statistically significant levels only after 30 min of exposure to LXA₄ (10⁻⁷ M). Similar results were obtained with ¹⁴C-labeled PA formation derived from ¹⁴C-arachidonate-labeled precursors. These findings indicated that LXA₄ can also stimulate other routes of PA formation in PMN but only after 30 min of exposure.

Only differentiated HL-60 cells (expressing specific binding sites for ³H-LXA₄) incubated with LXA₄ rapidly generated PEt that was evident within 30 sec. Undifferentiated HL-60 cells incubated with LXA₄ (10⁻⁹ M) did not rapidly generate PEt. The concentration dependence with these cells also gave a biphasic response with LXA₄ and gave an apparent maximum at 10⁻⁹ M. To investigate possible signal transduction events involved in LXA₄-mediated PLD activation, PMN and HL-60 cells were next exposed to either IAP or staurosporine. Results indicate that the LXA₄-mediated PLD activity evoked within the lower concentration range (10⁻⁹-10- ¹⁰ M) was sensitive to IAP treatment in both cell types and, similarly, the PLD activity stimulated at higher concentrations of LXA₄ (10⁻⁷ M) was inhibited by staurosporine. Thus, in both cellular systems at concentrations below 10⁻⁸ M, LXA₄ rapidly interacts with specific binding sites that trigger PLD activity and hence confers a functional response, while within submicromolar concentrations of LXA₄, it may stimulate additional processes that can also lead to activation of PLD.

### Example 4: Lipoxin Bioactivity Assays

Several of the preferred lipoxin analogs (shown structurally as compounds 1 through 8 above) were prepared by total synthesis as described in Example 1. Following preparation and isolation of these compounds via HPLC, compounds were first assessed to determine whether they retain biological activity using the neutrophil adhesion assay and epithelial cell transmigration assays (as described in Nash, S et al., (1987) J. Clin. Invest. 80:1104-1113; Nash, S et al., (1991) J. Clin. Invest. 87: 1474-1477; Parkos, C.A. et al., (1991) J. Clin. Invest. 88:1605-1612; Parkos, C.A. et al. (1992) J. Cell. Biol. 117:757-764; Madara J.L. et al., (1992) J. Tiss. Cult. Meth. 14:209-216).

Compounds 1 through 8 (10⁻⁷ - 10⁻¹⁰M) were found to inhibit neutrophil adhesion to endothelial cells and their transmigration on epithelial cells. The acetylenic precursors ( compound 1, 3, 5 and 7) were found to be physically more stable than their tetraene counterparts. Compound 7, which did not have an alcohol group in the C 15 position or other modifications in the series, showed no biological activity in the assays. It would therefore appear that a substituent in the C 15 position of lipoxin is necessary for the biological activity of at least lipoxin A₄ analogs. 15-methyl-lipoxin A₄ (compound 2) also proved to inhibit polymorphonuclear (PMN) adhesion triggered by leukotriene B₄ (LTB₄) to human endothelial cells with an IC₅₀ of ∼ 1 nM. Lipoxin analogs 1 through 8 were found to block migration at potencies greater than or equal to synthetic lipoxin A₄. Compound 7 was found to be essentially inactive within the concentration range for inhibition induced by LXA₄ or other analogs. The results in these neutrophil-containing bioassays indicate that lipoxin A₄ analogs with modifications in C15-C20 positions retain their biological action and can inhibit PMN transmigration and adhesion events.

The "bio-half-life" of compounds 1-8 was assessed using phorbol ester-treated human promyelocytic leukemia (HL-60) cells as described in Example 2. These cells converted more than 95% of lipoxin A₄ within five minutes of its addition to the cell incubation. Lipoxin A₄ in,this system was rapidly transformed to 15-oxo-lipoxin A₄. However, in the same assay, 15-methyl-lipoxin A₄ ( compound 2) and cyclohexyl-lipoxin A₄( compound 4) were quantitatively recovered in the incubation medium at times up to two hours. These results illustrate that modification in the carbon 20 through the carbon 15 positions prevents the further metabolism of lipoxin A₄ by leukocytes.

In addition, the stability of the acetylenic methyl ester lipoxin A₄ ( compound 1) was recovered essentially intact after 60 minutes of incubation in whole blood (37°C) ex vivo, as assessed after extraction and reverse phase HPLC. When taken together, these results indicate that lipoxin analogs retain biological action and are resistant to further metabolism in vitro.

### Equivalents

Those skilled in the art will recognize, or be able to ascertain using no more than routine experimentation, numerous equivalents to the specific procedures described herein. Such equivalents are considered to be within the scope of this invention and are covered by the following claims. Embodiments of the invention may include the features of the following enumerated paragraphs ("paras").
1. A lipoxin compound having an active region of a natural lipoxin and a metabolic transformation region resistant to in vivo metabolism.
2. A lipoxin compound of para 1, wherein the natural lipoxin is lipoxin A₄.
3. A lipoxin compound of para 1, wherein the natural lipoxin is lipoxin B₄
4. A lipoxin compound of para 2, which is not converted to a 15-oxo or 13-14-dihydro metabolite when administered to a subject in vivo or when cultured with monocytes in vitro.
5. A lipoxin compound of para 4 which binds to an LXA₄ specific receptor on differentiated HL-60 cells or leukocytes in a homologous displacement assay with a K_{d} comparable to or less than 0.6±0.3 nM.
6. A lipoxin compound of para 3, which is not converted to a 5-oxo or 6-7-dihydro metabolit when administered to a subject in vivo or when cultured with monocytes in vitro.
7. A lipoxin compound of the the structural formula: 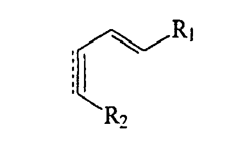 wherein R₁ can be 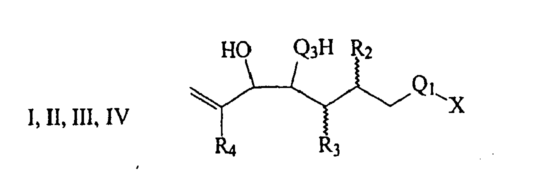 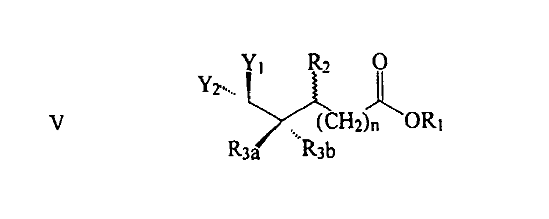 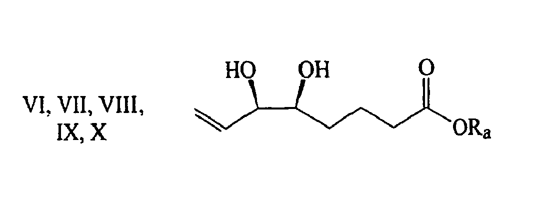 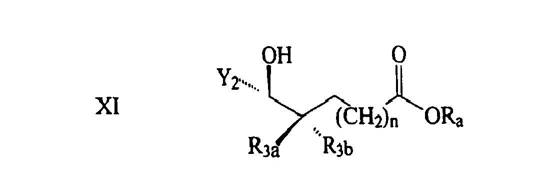 and R₂ can be 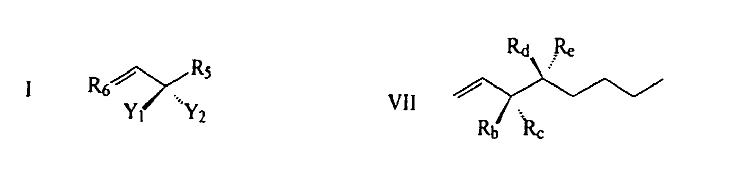 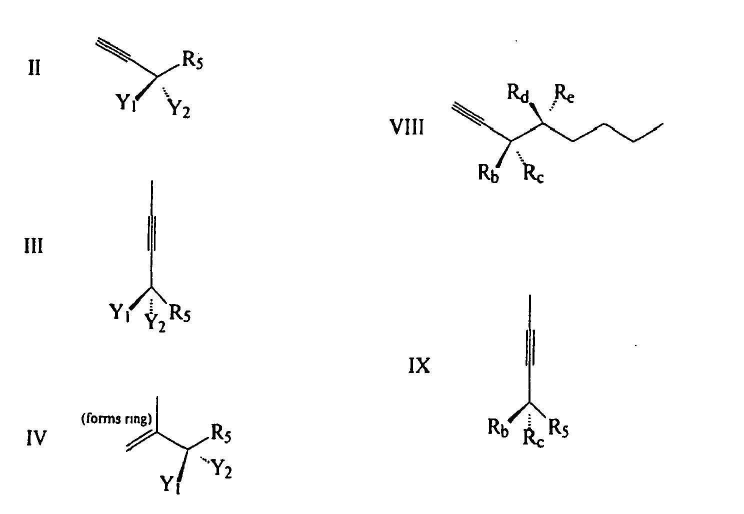 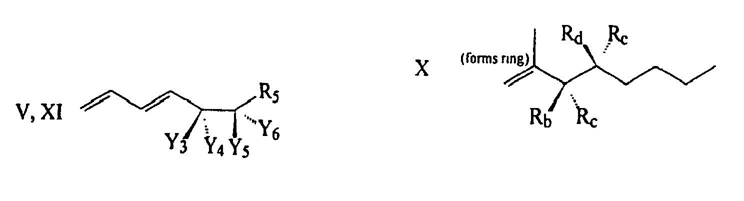 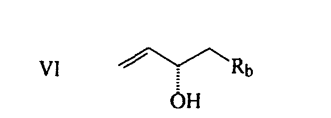
8. A lipoxin compound of the structural formula: 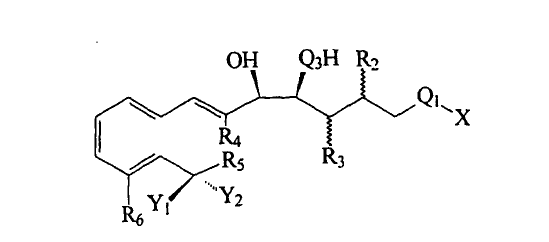 wherein X is R₁, OR₁, or SR₁;
   wherein R₁ is
   (i) hydrogen;
   (ii) alkyl of 1 to 8 carbons atoms, inclusive, which may be straight chain or branched;
   (iii) cycloalkyl of 3 to 10 carbon atoms, inclusive;
   (iv) aralkyl of 7 to 12 carbon atoms;
   (v) phenyl;
   (vi) substituted phenyl 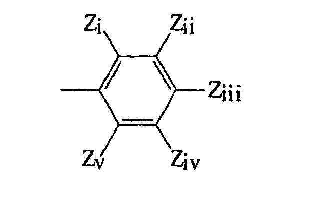 wherein Z ᵢ , Z ᵢᵢᵢ, and Zᵥ are each independently selected from the group consisting of -NO₂, -CN, -C(=O)-R₁, -SO₃H, and hydrogen;
      wherein Zᵢᵢ and Zᵢᵥ are each
      independently selected from the group consisting of halogen, methyl, hydrogen, and hydroxyl;
   (vii) detectable label molecule; or
   (viii) straight or branched chain alkenyl of 2 to 8 carbon atoms, inclusive;
   wherein Q₁ is (C=O), SO₂ or (CN);
   wherein Q₃ is O, S or NH;
   wherein one of R₂ and R₃ is hydrogen and the other is
   (a) H;
   (b) alkyl of 1 to 8 carbon atoms, inclusive, which may be straight chain or branched;
   (c) cycloalkyl of 3 to 6 carbon atoms, inclusive;
   (d) alkenyl of 2 to 8 carbon atoms, inclusive, which may be straight chain or branched; or
   (e) RₐQ₂R_{b}
      wherein Q₂ is -O- or -S-;
      wherein Rₐ is alkylene of 0 to 6 carbons atoms, inclusive, which may be straight chain or branched; and wherein R_{b} is alkyl of 0 to 8 carbon atoms, inclusive, which may be straight chain or branched;
   wherein R₄ is
   (a) H;
   (b) alkyl of 1 to 6 carbon atoms, inclusive, which may be straight chain or branched;
   wherein Y₁ or Y₂ is -OH , methyl, or -SH and wherein the other is
   (a) H
   (b) CHₐZ_{b}
      where a + b =3, a= 0 to 3, b = 0 to 3; and
      Z is cyano, nitro, or halogen;
   (c) alkyl of 2 to 4 carbon atoms, inclusive, straight chain or branched; or
   (d) alkoxy of 1 to 4 carbon atoms, inclusive;
   or Y₁ and Y₂ taken together are
   (a) =N; or
   (b) =O;
   wherein R₅ is
   (a) alkyl of 1 to 9 carbon atoms which may be straight chain or branched;
   (b)

      -(CH₂)ₙ -Rᵢ

      wherein n = 0 to 4 and Rᵢ is
      (i) cycloalkyl of 3 to 10 carbon atoms, inclusive;
      (ii) phenyl; or
      (iii) substituted phenyl 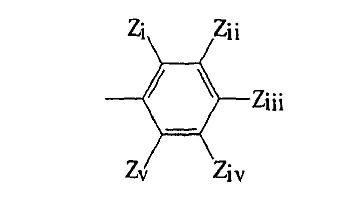 wherein Z ᵢ , Z ᵢᵢᵢ, and Zᵥ are each independently selected from the group consisting of hydrogen, -NO₂, -CN, -C(=O)-R₁, methoxy, and -SO₃H; and
         wherein Zᵢᵢ and Zᵢᵥ are each independently selected from the group consisting of halogen, methyl, hydrogen, and hydroxyl;
   (c)

      -RₐQₐR_{b}

      wherein Qₐ = -O- or -S-;
      wherein Rₐ is alkylene of 0 to 6 carbons atoms, inclusive, which may be straight chain or branched;
      wherein R_{b} is alkyl of 0 to 8 carbon atoms, inclusive, which may be straight chain or branched;
   (d)

      -C(Rᵢᵢᵢ)(Rᵢᵥ)-R₁

      wherein Rᵢᵢᵢ and Rᵢᵥ are selected independently from the group consisting of
      (i) H;
      (ii) CHₐZ_{b} where a + b = 3, a = 0 to 3, b = 0 + 3, and wherein any Z is independently selected from the group consisting of halogen;
   (e) haloalkyl of 1 to 8 carbon atoms, inclusive, and 1 to 6 halogen atoms, inclusive, straight chain or branched; and
   wherein R₆ is
   (a) H;
   (b) alkyl from 1 to 4 carbon atoms, inclusive, straight chain or branched;
   (c) halogen; but excluding the C-1 position amides, C-1 position alkanoates, and pharmaceutically acceptable C-1 position salts of (5S, 6R, 15S)-trihydroxy-7E, 9E, 11Z, 13E-eicosatetraenoic acid (LXA₄); and excluding C-5, C-6, and C-15 position alkanoates of LXA₄.
9. A compound of para 8 selected from the group consisting of: 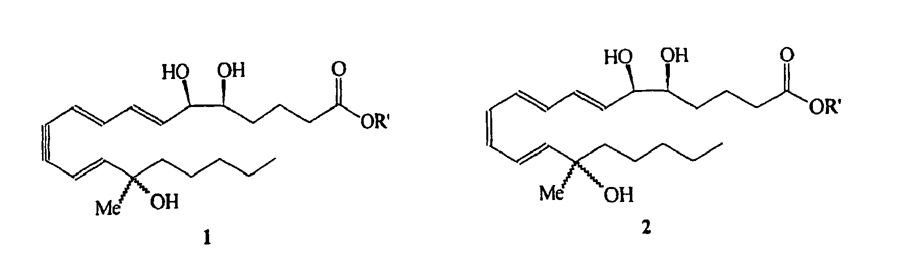 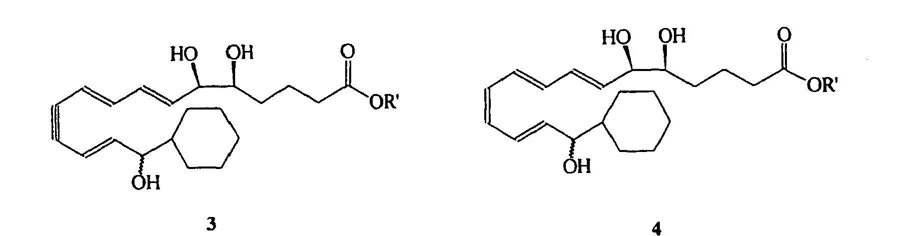 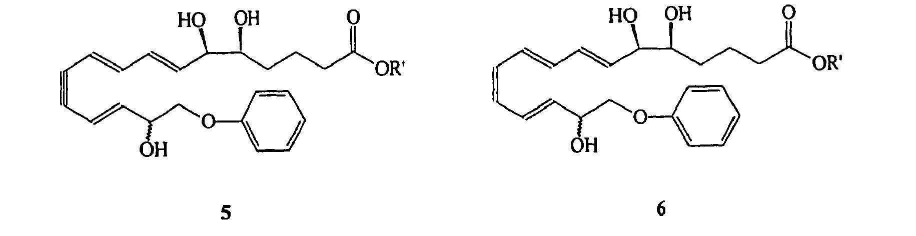 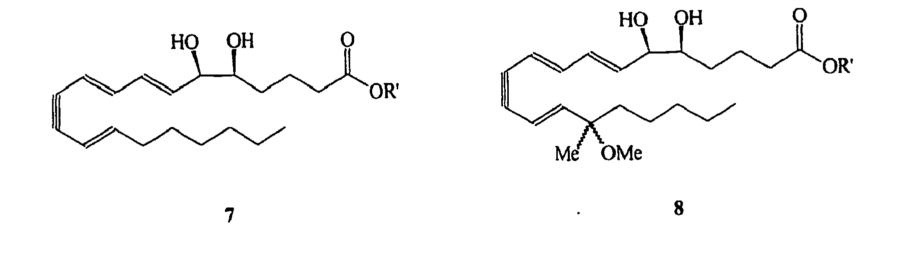 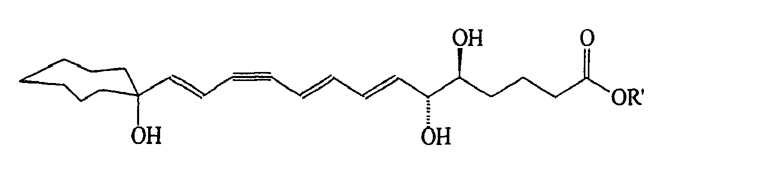 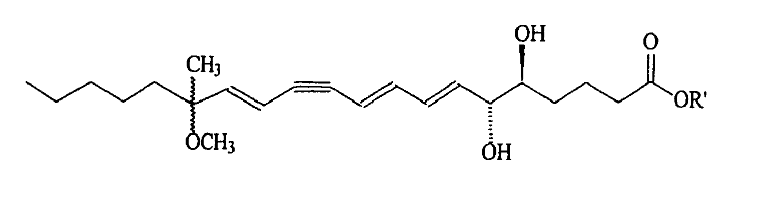 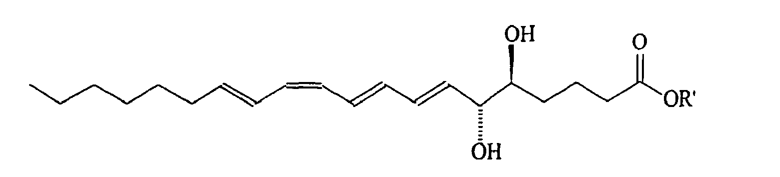 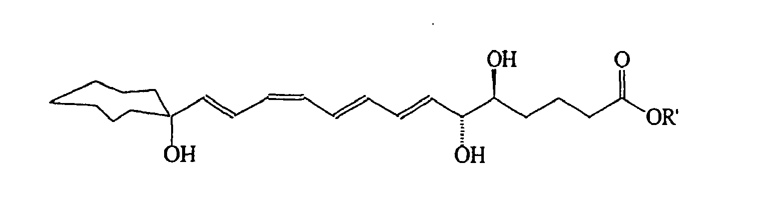 where R' is H or CH₃.
   10. A pharmaceutical composition comprising the compound of para 1 and a pharmaceutically acceptable carrier.
   11. A method for treating or preventing inflammation or an inflammatory response in the subject, comprising: administering to a subject an effective anti-inflammatory amount of a compound of para 10.
   12. A method of claim 11, wherein the inflammatory response results from the activation of leukocytes, which activation comprises leukocyte migration and generation of reactive oxygen species to evoke vascular leakage or edema.
   13. A method of para 12, wherein the inflammatory response is associated with rheumatoid arthritis, or asthma.
   14. A method of para 12, wherein the inflammatory response results from physical injury, including physical trauma and radiation exposure.
   15. A method of inducing vasodilation to treat or prevent a vasocontractive response or condition, comprising:
      administering to a subject an effective vasodilatory amount of a compound of para 10.
   16. A method of para 15, wherein the vasocontractive response or condition is selected from the group consisting of a renal hemodynamic disease, including glomerular disease, and a cardiovascular disease, including hypertension, myocardial infarction, and myocardial ischemia.
   17. A method for antagonizing a vasoconstrictive response to a sulfidopeptide leukotriene in a subject, comprising:
      administering to the subject a composition of para 10.
   18. A method of para 17, wherein the vasoconstrictive response to said leukotriene is associated with a medical disorder selected from the group consisting of: asthma, anaphylactic reactions, allergic reactions, shock, inflammation, rheumatoid arthritis, gout, psoriasis, allergic rhinitis, adult respiratory distress syndrome, Crohn's disease, endotoxin shock, traumatic shock, hemmorrhagic shock, bowel ischemic shock, renal glomerular disease, benign prostatic hypertrophy, inflammatory bowel disease, myocardial ischemia, myocardial infarction, circulatory shock, brain injury, systemic lupus erythematosus, chronic renal disease, cardiovascular disease, and hypertension.
   19. A method of para 18 wherein the vasoconstrictive response is a renal vasoconstrictive response, including mild vasoconstriction, such as chronic renal disease, and chronic severe vasoconstriction, such as glomerular kidney disease.
   20. A method for stimulating cell proliferation in a subject to treat or prevent myeloid suppressive disorders comprising: administering to the subject an effective amount of the compound of para 10.

## Claims

1. A lipoxin compound having an active region of a natural lipoxin and a metabolic transformation region resistant to in vivo metabolism, wherein the natural lipoxin is either lipoxin A4, or lipoxin B4.

2. A lipoxin compound according to claim 1, wherein the natural lipoxin is lipoxin A4, which is not converted to a 15-oxo or 13-14-dihydro metabolite when administered to a subject in vivo or when cultured with monocytes in vitro.

3. A lipoxin compound according to claim 2, which binds to an LXA₄ specific receptor on differentiated HL-60 cells or leukocytes in a homologous displacement assay with a K_{d} comparable to or less than 0.6 ± 0.3 nM.

4. A lipoxin compound according to claim 1, wherein the natural lipoxin is lipoxin B4, which is not converted to a 5-oxo or 6-7-dihydro metabolite when administered to a subject in vivo or when cultured with monocytes in vitro.
